# EUROPÄISCHE PATENTSCHRIFT

(11) **EP 0 912 089 B1**
(45) Veröffentlichungstag und Bekanntmachung des Hinweises auf die Patenterteilung: **28.11.2001**
(21) Anmeldenummer: 97922980.4
(22) Anmeldetag: 06.05.1997
(51) Int. Cl.: A01N 41/06, A01N 43/08, A01N 43/10, A01N 43/36, C07C 311/51, C07C 323/62, C07C 333/08, C07D 207/26, C07D 333/38, C07D 307/68

(54) **NEUE N-ACYLSULFONAMIDE, NEUE MISCHUNGEN AUS HERBIZIDEN UND ANTIDOTS UND DEREN VERWENDUNG**
NOVEL N-ACYL SULPHONAMIDES, NOVEL MIXTURES OF HERBICIDES AND ANTIDOTES AND THEIR USE
NOUVEAUX N-ACYLSULFONAMIDES, NOUVEAUX MELANGES D'HERBICIDES ET D'ANTIDOTES ET LEUR UTILISATION

(30) Priorität: 29.05.1996 DE 19621522
(43) Veröffentlichungstag der Anmeldung: 06.05.1999
(73) Patentinhaber: Aventis CropScience GmbH, 65929 Frankfurt am Main (DE)
(72) Erfinder: ZIEMER, Frank, D-65830 Kriftel (DE); HAAF, Klaus, D-65779 Kelkheim (DE); WILLMS, Lothar, D-65719 Hofheim (DE); BAUER, Klaus, D-63456 Hanau (DE); BIERINGER, Hermann, D-65817 Eppstein (DE); ROSINGER, Christopher, D-65719 Hofheim (DE)
(86) Internationale Anmeldenummer: EP9702305
(87) Internationale Veröffentlichungsnummer: WO9745016

(56) Entgegenhaltungen:
- EP-A- 0 365 484
- US-A- 2 411 495
- US-A- 2 423 976
- US-A- 2 503 820
- PROCEEDINGS OF THE INDIAN ACADEMY OF SCIENCES, SECTION A, Bd. 31, 1950, BANGALORE, IN, Seiten 117-123, XP002037438 C.V. DELIWALA, ET AL.: "Chemotherapy of tuberculosis. Part IV. Synthesis of phthalyl and ortho-toluoyl derivatives of the sulphonamides and sulphones as possible mycobacterial antagonists"
- JOURNAL OF THE INDIAN CHEMICAL SOCIETY, Bd. 22, 1945, CALCUTTA, IN, Seiten 343-347, XP002037441 J. SKIDAR, ET AL.: "Studies on sulphonamides and analogous compounds"
- JOURNAL OF THE INDIAN CHEMICAL SOCIETY, Bd. 24, 1947, CALCUTTA, IN, Seiten 173-176, XP002037442 B.C. JAIN, ET AL.: "Studies in sulphanilamides. Part XIII. Reaction with dicarboxylic acids. Some new N1- and N4-acyl and heterocyclic derivatives"
- JOURNAL OF THE INDIAN CHEMICAL SOCIETY, Bd. 24, 1947, CALCUTTA, IN, Seiten 466-468, XP002037439 U.P. BASU, ET AL.: "Aurothioacyl compounds from sulfa drugs"
- JOURNAL OF THE AMERICAN CHEMICAL SOCIETY, Bd. 65, November 1943, WASHINGTON, DC, US, Seiten 2126-2128, XP002037440 C. SIEBENMANN, ET AL.: "Chemotherapeutic study of p-nitrobenzoyl- and related compounds"
- ARCHIVES OF BIOCHEMISTRY AND BIOPHYSICS, Bd. 316, Nr. 1, 10.Januar 1995, Seiten 523-528, XP002037443 P.J. TUMMINO, ET AL.: "Competitive inhibition of HIV-1 protease by biphenyl carboxylic acids"
- CHEMICAL ABSTRACTS, vol. 46, no. 22, 25.November 1952 Columbus, Ohio, US; abstract no. 11154b, S. MARUYAMA, ET AL.: "Studies on antituberculotics. II. Preparation of N1-p-aminosalicylsulfanilide and N-p-aminosalicyl-p-phenolsulfonamide" Spalte 11154; XP002037444 & JOURNAL OF THE PHARMACEUTICAL SOCIETY OF JAPAN, Bd. 72, 1952, Seiten 596-599,
- PATENT ABSTRACTS OF JAPAN vol. 97, no. 3, 31.März 1997 & JP 08 286369 A (FUJI PHOTO FILM CO LTD), 1.November 1996,

## Beschreibung

Die Erfindung betrifft das technische Gebiet der Pflanzenschutzmittel, vorzugsweise der Safener gegen phytotoxische Nebenwirkungen von Pestiziden, insbesondere Herbziden, bei Kulturpflanzen; insbesondere betrifft sie Wirkstoff-Antidot-Kombinationen, die hervorragend für den Einsatz gegen konkurrierende Schadpflanzen in Nutzpflanzenkulturen geeignet sind.

Bei der Anwendung von Pflanzenbehandlungsmitteln, insbesondere von Herbiziden gegen Schadpflanzen in Pflanzenkulturen, können unerwünschte Schäden an den Kulturpflanzen auftreten. Insbesondere wenn die Herbizide mit den Kulturpflanzen nicht voll verträglich (selektiv) sind, lassen sich die Herbizide nur begrenzt verwenden. Sie können dann nicht oder nur in so geringen Aufwandmengen eingesetzt werden, daß die erwünschte breite herbizide Wirksamkeit nicht gewährleistet ist. Beispielsweise können viele Herbizide aus der Reihe der Sulfonylharnstoffe nicht selektiv in Mais, Reis oder anderem Getreide eingesetzt werden. Es ist daher wünschenwert, eine Phytotoxizität der Herbizide an den Kulturpflanzen möglichst zu vermeiden oder zu verringern. Verbindungen, welche geeignet sind, phytotoxische Nebenwirkungen von Herbiziden an Kulturpflanzen zu reduzieren werden Safener oder Antidots genannt.

Aus US-A-3498780 sind 1,4-substituierten Arylsulfonamide und deren herbizide Wirkung im Vorauflaufverfahren bekannt. US-A-4266078 beschreibt den Einsatz von N-Acylsulfonamiden als Safener für im Vorauflauf einzusetzende Thiocarbamat- und Halogenacetanilid-Herbizide; ein Einsatz als Safener für im Nachauflauf wirksame Herbizide ist daraus nicht bekannt. In US-A-4434000 werden ferner N-Benzolsulfonyl-carbamate als Safener für Harnstoff-Herbizide beschrieben. Aus EP-A-365484, EP-A-597807 und EP-A-600836 sind bereits N-Acylsulfamoylphenylharnstoffe und deren Anwendung als Safener für diverse Herbizidklassen bekannt.

Ganz unerwartet haben neue experimentelle Arbeiten gezeigt, daß N-(Acylsulfamoylphenyl)-alkanamide hervorragend dazu geeignet sind, phytotoxischen Nebenwirkungen von Pestiziden, bespielsweise von im Nachauflauf einsetzbaren Herbiziden, z. B. den als Acetolactatsynthase-hemmer (ALS-Hemmstoffe) wirkenden Sulfonylharnstoffen oder Imidazolinonen oder den Fettsäurebiosynthese-inhibitoren wie den (Hetero)Aryloxyphenoxycarbonsäure-derivaten, an Kulturpflanzen wie beispielsweise Mais, Reis oder anderem Getreide deutlich zu vermindern oder ganz aufzuheben.

Erfindungsgemäß einzusetzende Safenerwirkstoffe sind Verbindungen der Formel (I) und ihre Salze, 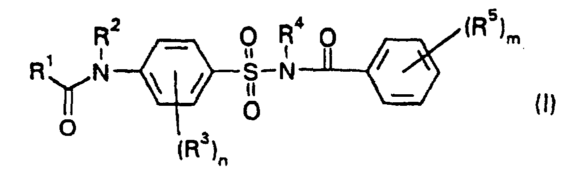 worin
- R¹: Wasserstoff, einen Kohlenwasserstoffrest, einen Kohlenwasserstoffoxyrest, einen Kohlenwasserstoffthiorest oder einen Heterocyclylrest, wobei jeder der letztgenannten 4 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a}-R^{a} substituiert ist, wobei jeder Kohlenwasserstoffteil vorzugsweise 1 bis 20 C-Atome aufweist und ein C-haltiger Rest R¹ inklusive Substituenten vorzugsweise 1 bis 30 C-Atome aufweist,
- R²: Wasserstoff oder (C₁-C₄)-Alkyl, vorzugsweise H, oder
- R¹ und R²: zusammen mit der Gruppe der Formel -CO-N- den Rest eines 3- bis 8-gliedrigen gesättigten oder ungesättigten Rings und
- R³: im Falle daß n = 1 ist, oder die R³ unabhängig voneinander, im Falle daß n größer als 1 ist, jeweils Halogen, Cyano, Nitro, Amino, Hydroxy, Carboxy, Formyl, CONH₂, SO₂NH₂ oder einen Rest der Formel -Z^{b}-R^{b},
- R⁴: Wasserstoff oder (C₁-C₄)-Alkyl, vorzugsweise H,
- R⁵: im Falle daß m=1 ist, oder die R⁵ unabhängig voneinander, im Falle daß m größer als 1 ist, jeweils Halogen, Cyano, Nitro, Amino, Hydroxy, Carboxy, CHO, CONH₂, SO₂NH₂ oder einen Rest der Formel -Z^{c}-R^{c},
- R^{a}: einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind,
- R^{b},R^{c}: unabhängig voneinander einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Phosphoryl, Halogen-(C₁-C₄)-alkoxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind,
- Z^{a}: eine divalente Gruppe der Formel -O-, -S-, -CO-, -CS-, -CO-O-, -CO-S-, -O-CO-, -S-CO-, -SO-, -SO₂-, -NR*-, -CO-NR*-, -NR*-CO-, -SO₂-NR*- oder -NR*-SO₂-, wobei die rechts angegebene Bindung der jeweiligen divalenten Gruppe die Bindung zum Rest R^{a} ist und wobei die R* in den letztgenannten 5 Resten unabhängig voneinander jeweils H, (C₁-C₄)-Alkyl oder Halo-(C₁-C₄)-alkyl bedeuten,
- Z^{b},Z^{c}: unabhängig voneinander eine direkte Bindung oder eine divalente Gruppe der Formel -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*- oder -NR*-CO-, wobei die rechts angegebene Bindung der jeweiligen divalenten Gruppe die Bindung zum Rest R^{b} bzw. R^{c} ist und wobei die R* in den letztgenannten 5 Resten unabhängig voneinander jeweils H, (C₁-C₄)-Alkyl oder Halo-(C₁-C₄)-alkyl bedeuten,
- n: eine ganze Zahl von 0 bis 4, vorzugsweise 0, 1 oder 2, insbesondere 0 oder 1, und
- m: eine ganze Zahl von 0 bis 5, vorzugsweise 0, 1, 2 oder 3, insbesondere 0, 1 oder 2, bedeuten.

In der Formel (I) und den im folgenden verwendeten Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 4 C-Atomen bzw. bei ungesättigten Gruppen mit 2 bis 4 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t-oder 2-Butyl, Pentyle, Hexyle, wie n-Hexyl, i-Hexyl und 1,3-Dimethylbutyl, Heptyle, wie n-Heptyle, 1-Methylhexyl und 1,4-Dimethylpentyl; Cycloalkyl bedeutet ein carbocyclisches gesättigtes Ringsystem, beispielsweise mit 3 bis 8 Ringatomen, z.B. Cyclopropyl, Cyclobutyl, Cyclopentyl, Cyclohexyl usw.; Alkenyl-, Alkinyl- und Cycloalkenylreste haben die Bedeutung der den Alkyl- bzw. Cycloalkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, Methyl-but-3-en-1-yl und 1-Methyl-but-2-en-1-yl; Cycloalkenyl ist beispielsweise Cyclopentenyl oder Cyclohexenyl; Alkinyl bedeutet z.B. Propargyl, But-2-in-1-yl, But-3-in-1-yl oder 1-Methyl-but-3-in-1-yl. Alkenyl in der Form "(C₃-C₄)-Alkenyl" oder "(C₃-C₆)-Alkenyl" bedeutet vorzugsweise einen Alkenylrest mit 3 bis 4 bzw. 3 bis 6 C-Atomen, bei dem die Doppelbindung nicht an dem C-Atom liegt, das mit dem übrigen Molekülteil der Verbindung (I) verbunden ist ("yl"-Position). Entsprechendes gilt für (C₃-C₄)-Alkinyl usw.

Halogen bedeutet beispielsweise Fluor, Chlor, Brom oder lod. Haloalkyl, -alkenyl und-alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl₂, CCl₃, CHCl₂, CH₂CH₂Cl; Haloalkyl ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl, vorzugsweise Alkyl, Alkenyl oder Alkinyl mit bis zu 12 C-Atomen oder Cycloalkyl mit 3 bis 6 Ringatomen oder Phenyl; entsprechendes gilt für einen Kohlenwasserstoffoxy- oder Kohlenwasserstoffthiorest.

Aryl bedeutet ein mono-, bi- oder polycyclisches aromatisches System, beispielsweise Phenyl, Naphthyl, Tetrahydronaphthyl, Indenyl; Indanyl, Pentalenyl, Fluorenyl und ähnliches, vorzugsweise Phenyl; Aryloxy bedeutet vorzugsweise ein dem genannten Arylrest entsprechender Oxy-Rest, insbesondere Phenoxy.
Heteroaryl oder ein heteroaromatischer Rest bedeutet ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl. Im substituierten Fall werden insbesondere auch bicyclische oder polycyclische aromatische oder mit cycloaliphatischen Ringen anellierte Verbindungen, z.B. Chinolinyl, Benzoxazolyl etc. eingeschlossen. Heteroaryl schließt auch einen heteroaromatischen Ring ein, der vorzugsweise 5- oder 6-gliedrig ist und 1,2-oder 3 Heteroringatome, insbesondere aus der Gruppe N, O und S enthält. Im substituierten Fall kann der heteroaromatische Ring auch benzokondensiert sein.

Ein heterocyclischer Rest (Heterocyclyl) oder Ring (Heterocyclus) kann gesättigt, ungesättigt oder heteroaromatisch sein; er enthält ein oder mehrere Heteroringatome, vorzugsweise aus der Gruppe N, O und S; vorzugsweise ist er ein aliphatischer Heterocyclylrest mit 3 bis 7 Ringatomen und bis zu 3 Heteroringatomen oder ist ein heteroaromatischer Rest mit 5 oder 6 Ringatomen und bis zu 3 Heteroringatomen. Der Rest kann z.B. ein wie oben definierter heteroaromatischer Rest oder Ring sein oder ist ein partiell hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Sind Substitutionen definiert durch "einen oder mehrere Reste aus einer Gruppe von Resten" beinhaltet dies sowohl die Substitution durch einen oder mehrere gleiche Reste als auch die einfache oder mehrfache Substitution durch unterschiedliche Reste.
Substituierte Reste, wie substituierte Kohlenwasserstoffreste, z.B. substitutiertes Alkyl, Alkenyl, Alkinyl, Aryl, Phenyl und Benzyl, oder substituiertes Heterocyclyl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, substituiertes Amino wie Acylamino, Mono- oder Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)-Alkyl, vorzugsweise Methyl oder Ethyl, (C₁-C₄)-Haloalkyl, vorzugsweise Trifluormethyl, (C₁-C₄)-Alkoxy, vorzugsweise Methoxy oder Ethoxy, (C₁-C₄)-Haloalkoxy, Nitro und Cyano. Besonders bevorzugt sind dabei die Substituenten Methyl, Methoxy und Chlor. Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)-Alkyl, (C₁-C₄)-Alkoxy, (C₁-C₄)-Halogenalkyl, (C₁-C₄)-Halogenalkoxy und Nitro substituiert ist, z.B. o-, m- und p-Tolyl, Dimethylphenyle, 2-, 3- und 4-Chlorphenyl, 2-, 3-und 4-Trifluor- und -Trichlorphenyl, 2,4-, 3,5-, 2,5- und 2,3-Dichlorphenyl, o-, m- und p-Methoxyphenyl.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)-Alkanoyl. Entsprechendes gilt für substituiertes Hydroxylamino oder Hydrazino.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-lsomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die Verbindungen der Formel (I) können Salze bilden, bei denen der Wasserstoff der -SO₂-NH-Gruppe, d. h. im Falle von R⁴ = H, oder auch andere acide Wasserstoffatome (z.B. aus COOH u.a.) durch ein für die Landwirtschaft geeignetes Kation ersetzt wird. Diese Salze sind beispielsweise Metallsalze; vorzugsweise Alkali- oder Erdalkalisalze, insbesondere Natrium- und Kaliumsalze, oder auch Ammoniumsalze oder Salze mit organischen Aminen. Ebenso kann Salzbildung durch Anlagerung einer Säure an basische Gruppen, wie z.B. Amino und Alkylamino, erfolgen. Geeignete Säuren hierfür sind starke anorganische und organische Säuren, beispielsweise HCI, HBr, H₂SO₄ oder HNO₃.

Aus Gründen der höheren Safenerwirkung und/oder besseren Herstellbarkeit sind erfindungsgemäße Verbindungen der Formel (I) oder deren Salze von näherem Interesse, worin
- R¹: Wasserstoff, (C₁-C₁₂)-Alkyl, (C₂-C₈)-Alkenyl, (C₂-C₈)-Alkinyl, (C₃-C₈)-Cycloalkyl, (C₃-C₈)-Cycloalkenyl, (C₁-C₈)-Alkoxy, (C₂-C₈)-Alkenyloxy, (C₂-C₈)-Alkinyloxy, (C₃-C₈)-Cycloalkoxy, (C₃-C₈)-Cycloalkenyloxy, (C₁-C₈)-Alkylthio, (C₂-C₈)-Alkenylthio, (C₂-C₈)-Alkinylthio, (C₃-C₈)-Cycloalkylthio, (C₃-C₈)-Cycloalkenylthio, Aryl oder Heterocyclyl mit 3 bis 8 Ringatomen und 1 bis 3 Heteroringatomen aus der Gruppe N, O und S, wobei jeder der letztgenannten 17 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a}-R^{a} substituiert ist,
- R²: Wasserstoff oder (C₁-C₄)-Alkyl, vorzugsweise H, oder
- R¹ und R²: zusammen mit der Gruppe der Formel -CO-N- den Rest eines 3- bis 8-gliedrigen gesättigten oder ungesättigten heterocyclischen Rings, der neben dem N-Atom der Gruppe der Formel -CO-N- noch 1 oder 2 Heteroatome aus der Gruppe N, O und S enthalten kann, und
- R³,: im Falle daß n=1 ist, oder die R³ unabhängig voneinander, im Falle daß n größer als 1 ist, jeweils Halogen, Cyano, Nitro, Amino, Hydroxy, Phosphoryl, Carboxy, CHO, CONH₂, SO₂NH₂ oder einen Rest der Formel -Z^{b}-R^{b},
- R⁴: Wasserstoff oder (C₁-C₄)-Alkyl, vorzugsweise H,
- R⁵,: im Falle daß m =1 ist, oder die R⁵ unabhängig voneinander, im Falle daß m größer als 1 ist, jeweils Halogen, Cyano, Nitro, Amino, Hydroxy, Carboxy, Phosphoryl, CHO, CONH₂ SO₂NH₂ oder einen Rest der Formel -Z^{c}-R^{c},
- R^{a}: (C₁-C₈)-Alkyl, (C₃-C₆)-Cycloalkyl, (C₂-C₈)-Alkenyl, (C₃-C₆)-Cycloalkenyl, (C₂-C₈)-Alkinyl, Phenyl oder einen Heterocyclyirest mit 3 bis 6 Ringatomen und 1 bis 3 Heteroringatomen aus der Gruppe N, O und S, wobei jeder der 7 letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind,
- R^{b},R^{c}: unabhängig voneinander (C₁-C₈)-Alkyl, (C₃-C₆)-Cycloalkyl, (C₂-C₈)-Alkenyl, (C₃-C₆)-Cycloalkenyl, (C₂-C₈)-Alkinyl, Phenyl oder einen Heterocyclylrest mit 3 bis 6 Ringatomen und 1 bis 3 Heteroringatomen aus der Gruppe N, O und S, wobei jeder der 7 letztgenannten Reste unsubstituiert oder durch einen oder mehrere gleiche oder verschiedene Reste aus der Gruppe Halogen, Cyano, Nitro, Amino, Hydroxy, Phosphoryl, Halogen-(C₁-C₄)-alkoxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind,
- Z^{a}: eine divalente Gruppe der Formel -O-, -S-, -CO-, -CS-, -CO-O-, -CO-S-, -O-CO-, -S-CO-, -SO-, -SO₂-, -NR*-, -CO-NR*- oder -NR*-CO-, wobei die rechts angegebene Bindung der jeweiligen divalenten Gruppe die Bindung zum Rest R^{a} ist und wobei die R* in den letztgenannten beiden Resten unabhängig voneinander jeweils H, (C₁-C₄)-Alkyl oder (C₁-C₄)-Haloalkyl bedeuten,
- Z^{b},Z^{c}: unabhängig voneinander eine direkte Bindung oder eine divalente Gruppe der Formel -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*- oder -NR*-CO-, wobei die rechts angegebene Bindung der jeweiligen divalenten Gruppe die Bindung zum Rest R^{b} bzw. R^{c} ist und wobei die R* in den letztgenannten 5 Resten unabhängig voneinander jeweils H, (C₁-C₄)-Alkyl oder Halo-(C₁-C₄)-Alkyl bedeuten,
bedeuten.

Von besonderem Interesse sind erfindungsgemäße Safener der Formel (I) oder deren Salze, worin
- R¹: Wasserstoff, (C₁-C₁₂)-Alkyl, (C₂-C₈)-Alkenyl, (C₂-C₈)-Alkinyl, (C₃-C₈)-Cycloalkyl, (C₃-C₈)-Cycloalkenyl, (C₁-C₈)-Alkoxy, (C₂-C₈)-Alkenyloxy, (C₂-C₈)-Alkinyloxy, (C₃-C₈)-Cycloalkoxy, (C₃-C₈)-Cycloalkenyloxy, (C₁-C₆)-Alkylthio, (C₂-C₈)-Alkenylthio, (C₂-C₈)-Alkinylthio, (C₃-C₈)-Cycloalkylthio, (C₃-C₈)-Cycloalkenylthio, Phenyl oder Heterocyclyl mit 3 bis 6 Ringatomen und 1 bis 3 Heteroringatomen aus der Gruppe N, O und S, wobei jeder der vorstehenden C-haltigen Reste unsubstituiert oder durch einen oder mehrere, vorzugsweise bis zu drei gleiche oder verschiedene Substituenten aus der Gruppe Halogen, Nitro, Cyano, Amino, Hydroxy, (C₁-C₈)-Alkoxy, worin eine oder mehrere, vorzugsweise bis zu drei nicht benachbarte CH₂-Gruppen durch Sauerstoff ersetzt sein können, (C₁-C₈)-Alkylthio, (C₁-C₆)-Alkylsulfinyl, (C₁-C₆)-Alkylsulfonyl, (C₂-C₈)-Alkenyloxy, (C₂-C₈)-Alkenylthio, (C₂-C₈)-Alkinyloxy, (C₂-C₈)-Alkinylthio, (C₃-C₇)-Cycloalkyl, (C₃-C₇)-Cycloalkenyl, (C₃-C₇)-Cycloalkoxy, (C₃-C₇)-Cycloalkenyloxy, Mono- und Di-[(C₁-C₄)-alkyl]-amino, [(C₁-C₈)-Alkoxy]-carbonyl, [(C₂-C₈)-Alkenyloxy]-carbonyl, [(C₂-C₈)-Alkinyloxy]-carbonyl, [(C₁-C₈)-Alkylthio]-carbonyl, [(C₁-C₈)-Alkyl]-carbonyl, [(C₂-C₈)-Alkenyl]-carbonyl, [(C₂-C₈)-Alkinyl]-carbonyl, Phenyl, Phenyl-(C₁-C₆)-alkoxy, Heterocyclyl mit 3 bis 6 Ringatomen und 1 bis 3 Heteroringatomen aus der Gruppe N, O und S und im Falle cyclischer Reste auch (C₁-C₆)-Alkyl substituiert sind, wobei jeder der 25 letztgenannten Reste unsubstituiert oder durch einen oder mehrere Reste aus der Gruppe Halogen, Nitro, Amino, Cyano und Hydroxy substituiert ist,
- R²: Wasserstoff oder (C₁-C₄)-Alkyl, vorzugsweise H, oder
- R¹ und R²: zusammen mit der Gruppe der Formel -CO-N- den Rest eines 5- bis 6-gliedrigen gesättigten oder ungesättigten heterocyclischen Rings, der neben dem N-Atom der Gruppe der Formel -CO-N- noch 1 Heteroatom aus der Gruppe N, O und S enthalten kann, und
- R³, R⁵: jeweils gleiche oder verschiedene Reste, welche unabhängig voneinander Halogen, Nitro, Amino, Hydroxy, Cyano, Sulfamoyl, (C₁-C₈)-Alkyl, (C₃-C₆)-Cycloalkyl, (C₂-C₈)-Alkenyl, (C₂-C₈)-Alkinyl, (C₁-C₈)-Alkoxy, (C₂-C₈)-Alkenyloxy, (C₂-C₈)-Alkinyloxy, Mono- oder Di-[(C₁-C₄)-Alkyl]-aminosulfonyl, (C₁-C₈)-Alkylthio, (C₁-C₈)-Alkylsulfinyl, (C₁-C₈)-Alkylsulfonyl, (C₁-C₈)-Alkoxycarbonyl, (C₁-C₈)-Alkylthiocarbonyl, (C₁-C₈)-Alkylcarbonyl, wobei jeder der letztgenannten 15 Reste unsubstituiert oder durch einen oder mehrere, vorzugsweise bis zu drei gleiche oder verschiedene Reste aus der Gruppe Halogen, Halogen-(C₁-C₆)-alkoxy, Phosphoryl, Nitro, Amino, Cyano, Hydroxy, (C₁-C₈)-Alkoxy, worin eine oder mehrere, vorzugsweise bis zu drei nicht benachbarte CH₂-Gruppen durch Sauerstoff ersetzt sein können, und im Falle cyclischer Reste auch (C₁-C₄)-Alkyl und (C₁-C₄)-Haloalkyl substituiert ist,
bedeuten.

Von besonderem Interesse sind erfindungsgemäße Safener der Formel (I) oder deren Salze, worin
- R¹: Wasserstoff, (C₁-C₈)-Alkyl, (C₂-C₆)-Alkenyl, (C₂-C₆)-Alkinyl, (C₃-C₆)-Cycloalkyl, (C₅-C₆)-Cycloalkenyl, (C₁-C₆)-Alkoxy, (C₂-C₆)-Alkenyloxy, (C₂-C₆)-Alkinyloxy, (C₃-C₆)-Cycloalkoxy, (C₅-C₆)-Cycloalkenyloxy, (C₁-C₆)-Alkylthio, (C₂-C₆)-Alkenylthio, (C₂-C₆)-Alkinylthio, (C₃-C₆)-Cycloalkylthio, (C₅-C₆)-Cycloalkenylthio, Phenyl oder Heterocyclyl mit 3 bis 6 Ringatomen und 1 bis 3 Heteroringatomen aus der Gruppe N, O und S, wobei jeder der 17 letztgenannten Reste unsubstituiert oder durch einen oder mehrere gleiche oder verschiedene Substituenten aus der Gruppe Halogen, Cyano, (C₁-C₆)-Alkoxy, (C₁-C₆)-Alkylthio, (C₁-C₄)-Alkylsulfinyl, (C₁-C₄)-Alkylsulfonyl, (C₂-C₄)-Alkenyloxy, (C₂-C₄)-Alkenylthio, (C₂-C₄)-Alkinyloxy, (C₂-C₄)-Alkinylthio, (C₃-C₆)-Cycloalkyl, (C₅-C₆)-Cycloalkenyl, (C₃-C₆)-Cycloalkoxy, (C₅-C₆)-Cycloalkenyloxy, Mono- und Di-[(C₁-C₄)-alkyl]-amino, [(C₁-C₆)-Alkoxy]-carbonyl, [(C₁-C₆)-Alkylthio]-carbonyl, [(C₁-C₆)-Alkyl]-carbonyl, Phenyl, Phenyl-(C₁-C₄)-alkoxy, Heterocyclyl mit 5 bis 6 Ringatomen und 1 bis 3 Heteroringatomen aus der Gruppe N, O und S und im Falle cyclischer Reste auch (C₁-C₄)-Alkyl substituiert ist, wobei jeder der 21 letztgenannten Reste unsubstituiert oder durch einen oder mehrere Reste aus der Gruppe Halogen und Cyano und im Falle cyclischer Reste auch (C₁-C₄)-Alkyl substituiert ist,
- R²: Wasserstoff oder (C₁-C₄)-Alkyl oder
- R¹ und R²: zusammen mit der Gruppe der Formel -CO-N- den Rest eines 5- bis 6-gliedrigen gesättigten oder ungesättigten heterocyclischen Rings, der neben dem N-Atom der Gruppe der Formel -CO-N- kein weiteres Heteroringatom enthält, und
- R³, R⁵: jeweils gleiche oder verschiedene Reste, welche unabhängig voneinander Halogen, Nitro, Amino, Hydroxy, Cyano, Sulfamoyl, (C₁-C₆)-Alkyl, (C₃-C₆)-Cycloalkyl, (C₂-C₆)-Alkenyl, (C₂-C₆)-Alkinyl, (C₁-C₆)-Alkoxy, (C₂-C₆)-Alkenyloxy, (C₂-C₆)-Alkinyloxy, Mono- und Di-[(C₁-C₄)-Alkyl]-aminosulfonyl, (C₁-C₆)-Alkylthio, (C₁-C₈)-Alkylsulfinyl, (C₁-C₆)-Alkylsulfonyl, (C₁-C₆)-Alkoxycarbonyl, (C₁-C₆)-Alkylthiocarbonyl, (C₁-C₆)-Alkylcarbonyl, wobei jeder der letztgenannten 15 Reste unsubstituiert oder durch einen oder mehrere gleiche oder verschiedene Reste aus der Gruppe Halogen, Halogen-(C₁-C₄)-alkoxy, Cyano, (C₁-C₆)-Alkoxy und im Falle cyclischer Reste auch (C₁-C₄)-Alkyl und (C₁-C₄)-Haloalkyl substituiert ist,
bedeuten.

Bevorzugt als Safener sind solche Verbindungen (I) oder deren Salze, worin in der Formel (I)
- R¹: Wasserstoff, (C₁-C₆)-Alkyl, (C₃-C₆)-Cycloalkyl, (C₂-C₆)-Alkenyl, (C₅-C₆)-Cycloalkenyl, (C₁-C₆)-Alkoxy, Phenyl oder Heterocyclyl mit 3 bis 6 Ringatomen und 1 bis 3 Heteroringatomen, vorzugsweise 1 oder 2 Heteroringatomen, aus der Gruppe N, 0 und S, wobei jeder der 7 letztgenannten Reste unsubstituiert oder durch einen oder mehrere gleiche oder verschiedene Reste aus der Gruppe Halogen, (C₁-C₆)-Alkoxy, worin eine oder mehrere CH₂-Gruppen durch Sauerstoff ersetzt sein können, (C₁-C₆)-Halogenalkoxy, (C₁-C₂)-Alkylsulfinyl, (C₁-C₂)-Alkylsulfonyl, (C₃-C₆)-Cycloalkyl, (C₁-C₄)-Alkoxycarbonyl, (C₁-C₄)-Alkylcarbonyl und Phenyl und im Falle im Falle cyclischer Reste auch (C₁-C₄)-Alkyl und (C₁-C₄)-Haloalkyl substituiert ist,
- R²: Wasserstoff,
- R³: Halogen, Halogen-(C₁-C₄)-alkyl, Halogen-(C₁-C₄)-alkoxy, (C₁-C₄)-Alkyl, (C₁-C₄)-Alkoxy, (C₁-C₄)-Alkylsulfonyl, (C₁-C₄)-Alkoxycarbonyl oder (C₁-C₄)-Alkylcarbonyl,
- R⁴: Wasserstoff,
- R⁵: Halogen, (C₁-C₄)-Alkyl, (C₁-C₄)-Halogenalkyl, Halogen-(C₁-C₄)-alkoxy, (C₃-C₆)-Cycloalkyl, Phenyl, (C₁-C₄)-Alkoxy, Cyano, (C₁-C₄)-Alkylthio, (C₁-C₄)-Alkylsulfinyl, (C₁-C₄)-Alkylsulfonyl, (C₁-C₄)-Alkoxycarbonyl oder (C₁-C₄)-Alkylcarbonyl,
- n: 0, 1 oder 2 und
- m: 1 oder 2 bedeuten.

Besonders bevorzugt sind als Safener erfindungsgemäße Verbindungen der Formel (I) oder deren Salze, worin
- R¹: Wasserstoff, (C₁-C₆)-Alkyl, (C₃-C₆)-Cycloalkyl, Furanyl oder Thienyl, wobei jeder der letztgenannten 4 Reste unsubstituiert oder durch einen oder mehrere Substituenten aus der Gruppe Halogen, (C₁-C₄)-Alkoxy, Halogen-(C₁-C₆)-alkoxy und (C₁-C₄)-Alkylthio und im Falle cyclischer Reste auch (C₁-C₄)-Alkyl und (C₁-C₄)-Haloalkyl substituiert ist,
- R²: Wasserstoff,
- R³: Halogen, Halogen-(C₁-C₄)-alkyl, Halogen-(C₁-C₄)-alkoxy, (C₁-C₄)-Alkyl, (C₁-C₄)-Alkoxy, (C₁-C₄)-Alkylsulfonyl, (C₁-C₄)-Alkoxycarbonyl oder (C₁-C₄)-Alkylcarbonyl,
vorzugsweise Halogen, (C₁-C₄)-Halogenalkyl, wie Trifluormethyl, (C₁-C₄)-Alkoxy, Halogen-(C₁-C₄)-alkoxy, (C₁-C₄)-Alkoxycarbonyl oder (C₁-C₄)-Alkylsulfonyl,
- R⁴: Wasserstoff,
- R⁵: Halogen, (C₁-C₄)-Alkyl, Halogen-(C₁-C₄)-alkyl, Halogen-(C₁-C₄)-alkoxy, (C₃-C₆)-Cycloalkyl, Phenyl, (C₁-C₄)-Alkoxy, Cyano, (C₁-C₄)-Alkylthio, (C₁-C₄)-Alkylsulfinyl, (C₁-C₄)-Alkylsulfonyl, (C₁-C₄)-Alkoxycarbonyl oder (C₁-C₄)-Alkylcarbonyl,
vorzugsweise Halogen, (C₁-C₄)-Alkyl, (C₁-C₄)-Halogenalkyl, wie Trifluormethyl, Halogen-(C₁-C₄)-alkoxy, (C₁-C₄)-Alkoxy oder (C₁-C₄)-Alkylthio,
- n: 0, 1 oder 2 und
- m: 1 oder 2 bedeuten.

Die Verbindungen der Formel (I) sind zum Teil bekannt. Ihre Safenerwirkung ist jedoch noch nicht bekannt gewesen; siehe US-A-2411495, US-A-2423976, US-A-2503820, CH-242291, Bull. Chem. Soc. Jpn. 61 (1988) 3999-4003, vgl. a4; Chem. Abstr. 55: 461g; Chem. Abstr. 104: 125043; Chem. Abstr. 52: 9206i bis 9207a; Seydel et al., Arzneimittelforschung 14 (1964) 705, worin einige Verbindungen (I) als Zwischenprodukte für pharmazeutische Sulfonamide beschrieben sind. Bekannt sind Verbindungen der Formel (I), worin
a) R² =H, n = 0 und
   a1)
      R¹ = CH₃ und
      m = 0 oder (R⁵)ₘ =2-, 3- oder 4-CH₃, 4-C₂H₅, 4-n-C₃H₇, 4-i-C₃H₇, 4-OCH₃, 4-i-OC₃H₇, 4-NH₂, 4-Cl, 4-NO₂, 2,3-(CH₃)₂, 2,4-(CH₃)₂, 3,4-(CH₃)₂, 2,5-(CH₃)₂, 2,4,5-(CH₃)₃, 2,4,6-(CH₃)₃, 2,3,4,5,6-(CH₃)₅, 3-CH₃-4-OCH₃, 3-CH₃-4-SCH₃, 2,4-(OCH₃)₂, 2,5-(OCH₃)₂, 3,4,5-(OCH₃)₃, 2-OCH₃-4-NH₂, 2-OCH₃-4-NO₂ oder zwei Reste R³ zusammen die Gruppe -OCH₂O-,
   a2)
      R¹ = H, n-C₃H₇, n-C₆H₁₃, Cyclohexyl oder 2-Methylphenyl und
      (R⁵)ₘ = 2-CH₃,
   a3)
      R¹ = n-C₅H₁₁ und
      m = 0 oder (R⁵)ₘ = 2-CH₃, 3-NO₂, 4-NO₂, 2,3-(CH = CH-CH=CH),
   a4) R¹ = n-C₉H₁₉ und m = 0,
   a5) R¹ = OCH₃, (R⁵)ₘ =2-i-OC₃H₇,
   a6) R¹ = OC₂H₅, (R⁵)ₘ = 2-OCH₃, 2-COOH, 3,5-(CH₃)₂,
   a7)
      R¹ = CH₂CH₂COOH und
      m = O oder (R⁵)ₘ= 4-i-OC₃H₇,
   a8) R¹ = CH=CHCOOH und (R⁵)ₘ= 2-CH₃ oder 4-i-OC₃H₇,
   a9) R¹ = 4-Methoxyphenyl und (R⁵)ₘ= 4-OCH₃,
   a10) R¹ = 4-Nitrophenyl und (R⁵)ₘ= 4-NO₂,
   a11) R¹ = Benzdioxol-6-yl und (R⁵)ₘ = 3,4-(-OCH₂O-),
   a12)
      R¹ = 3,5-Dimethyl-1-phenyl-pyrazol-4-yl oder 2,3-Dimethyl-1-phenyl-5-oxo-pyrazol-4-yl und
      (R⁵)ₘ= 4-i-OC₃H₇,
   a13)
      R¹ = C₁₁H₂₃, CH₂Cl, CH₂Br, CH₂I, CHCl₂, CCl₃ oder CH₂F und
      (R⁵)ₘ = 3,4-(CH₃)₂; siehe Chem. Abstr, 55: 461g; oder
b) R¹ =H, R² = H, R⁴ = CH₃, n = m=0,
c) R¹ =CH₃, R² =H, (R³)ₙ= einen annellierten Benzolring in 2,3-Stellung und m = 0 oder
d) R¹ = Phenyl, R² = R⁴ = H, (R³)ₙ= 3-Phenylcarbonyloxy und m= 0
bedeuten.

Die Erfindung betrifft auch pestizide Mittel mit einem wirksamen Gehalt an
A) einem oder mehreren pestiziden Wirkstoffen,
B) einem oder mehreren erfindungsgemäßen Safenern der Formel (I) oder deren Salzen.

Als pestizide Wirkstoffe kommen beispielsweise Herbizide, Insektizide, Fungizide Acarizide und Nematizide, welche jeweils bei alleiniger Anwendung phytotoxische Schäden an den Kulturpflanzen ergeben, in Frage. Von besonderem Interesse sind entsprechende Pestizidwirkstoffe aus der Gruppe der Herbizide.

Bevorzugt sind herbizide Mittel mit einem Gehalt an
A) mindestens einem herbiziden Wirkstoff aus der Gruppe, welche ALS-Hemmstoffe und Fettsäurebiosynthese-inhibitoren enthält, und
B) mindestens einem erfindungsgemäßen Safener der Formel (I) oder dessen Salz.

Die Erfindung betrifft auch ein Verfahren zum Schutz von Kulturpflanzen, vorzugsweise Getreidepflanzen wie Mais, Reis, Weizen und Gerste, vor phytotoxischen Nebenwirkungen von Herbiziden, insbesondere aus der Gruppe der Sulfonylharnstoffe, das dadurch gekennzeichnet ist, daß eine wirksame Menge einer oder mehrerer Verbindungen der Formel (I) vor, nach oder gleichzeitig mit dem obengenannten herbiziden Wirkstoff auf die Pflanzen, Pflanzensamen oder die Anbaufläche appliziert wird.

Die Erfindung betrifft weiterhin die Verwendung von Verbindungen der Formel (I) zum Schutz von Kulturpflanzen vor phytotoxischen Nebenwirkungen der oben definierten Herbizide.

Die Verbindungen der allgemeinen Formel (I) und ihre Vorprodukte lassen sich nach allgemein bekannten Verfahren herstellen [z.B. K. Kojima et al., J. Pharm. Soc. Jpn., 71 (1951), 626; A.D.B. Sloan, Chem. Ind., 1969, 1305; Bretschneider et al., Monatsh. Chemie 87, (1956), 47; K. Takatori et al., J. Pharm. Soc. Jpn., 78, (1958), 546].

So kann die Herstellung der erfindungsgemäßen Verbindungen der Formel I in der Weise erfolgen, daß man
1. eine Verbindung der Formel (II), 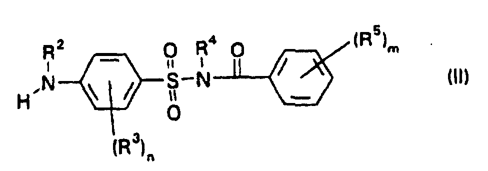 worin R², R³, R⁴, R⁵, n und m wie in Formel (I) definiert sind, mit einem Acylierungsmittel der Formel R¹-CO-Nuc, worin Nuc eine Abgangsgruppe ist, z.B. mit einem Acylierungsmittel wie einem Carbonsäurehalogenid oder oder Carbonsäureanhydrid R¹-CO-Cl bzw R¹-CO-O-CO-R¹ umsetzt, worin R¹ wie in Formel (I) definiert ist;
2. eine Verbindung der Formel (III), 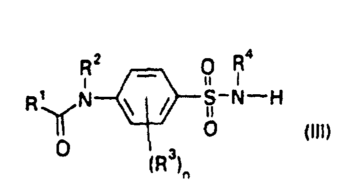 worin R¹, R², R³, R⁴ und n wie in Formel (I) definiert sind, mit einem Benzoylhalogenid der Formel (IV) umsetzt, 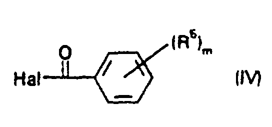 worin R⁵ und m die in Formel (I) gegebenen Bedeutungen haben.

Die Umsetzungen nach Varianten 1 und 2 erfolgen vorzugsweise in einem inerten organischen Lösungsmittel in Gegenwart eines säurebindenden Mittels. Als Lösungsmittel eignen sich beispielsweise aprotisch polare Lösungsmittel, z.B. Ether wie THF (Tetrahydrofuran) oder Dioxan, Ketone wie Acetonitril, Amide wie DMF (Dimethylformamid). Als Basen werden vorzugsweise organische Basen, z.B. substituierte Amine wie Triethylamin, Pyridin oder DMAP (Dimethylaminopyridin), verwendet.
Die Reaktionstemperaturen liegen vorzugsweise im Bereich zwischen -20 °C und 120 °C.

Werden die erfindungsgemäßen Safener der Formel (I) in subtoxischen Konzentrationen zusammen mit den herbiziden Wirkstoffen oder auch in einer beliebigen Reihenfolge ausgebracht, so sind sie in der Lage, die phytotoxischen Nebenwirkungen dieser Herbizide zu reduzieren bzw. völlig aufzuheben, ohne jedoch die Wirksamkeit der Herbizide gegenüber den Schadpflanzen zu vermindern. Geeignete Herbizide, die mit den erfindungsgemäßen Safenern kombiniert werden können, sind beispielsweise solche aus der Gruppe der Sulfonylharnstoffe, der Imidazolinone, der (Hetero)Aryloxy-phenoxyalkancarbonsäurederivate, der Cyclohexandione, der Benzoylcyclohexandione, der Triazolopyrimidin-sulfonamide, der Pyrimidinyloxy-pyrimidincarbonsäure- und -benzoesäurederivate und der S-(N-Aryl-N-alkylcarbamoylmethyl)dithiophosphorsäureester.

Geeignete Herbizide aus der Sulfonylharnstoff-Reihe sind z. B. Pyrimidinyl- oder Triazinyl-aminocarbonyl-[benzol-, pyridin-, pyrazol-, thiophen- und (alkylsulfonyl)alkylamino-]-sulfamide. Bevorzugt als Substituenten am Pyrimidinring oder Triazinring sind Alkoxy, Alkyl, Haloalkoxy, Haloalkyl, Halogen oder Dimethylamino, wobei alle Substituenten unabhängig voneinander kombinierbar sind. Bevorzugte Substituenten im Benzol-, Pyridin-, Pyrazol-, Thiophen- oder (Alkylsulfonyl)alkylamino-Teil sind Alkyl, Alkoxy, Halogen, Nitro, Alkoxycarbonyl, Aminocarbonyl, Alkylaminocarbonyl, Dialkylaminocarbonyl, Alkoxyaminocarbonyl, Halogenalkoxy, Halogenalkyl, Alkylcarbonyl, Alkoxyalkyl, (Alkansulfonyl)alkylamino. Geeignete Sulfonylharnstoffe sind beispielsweise
1) Phenyl- und Benzylsulfonylharnstoffe und verwandte Verbindungen, z. B.
   1-(2-Chlorphenylsulfonyl)-3-(4-methoxy-6-methyl-1,3,5-triazin-2-yl)harnstoff (Chlorsulfuron),
   1-(2-Ethoxycarbonylphenylsulfonyl)-3-(4-chlor-6-methoxypyrimidin-2-yl)harnstoff (Chlorimuron-ethyl),
   1-(2-Methoxyphenylsulfonyl)-3-(4-methoxy-6-methyl-1,3, 5-triazin-2-yl)-harnstoff (Metsulfuron-methyl),
   1-(2-Chlorethoxyphenylsulfonyl)-3-(4-methoxy-6-methyl-1,3,5-triazin-2-yl)-harnstoff (Triasulfuron),
   1-(2-Methoxycarbonylphenylsulfonyl)-3-(4,6-dimethylpyrimidin-2-yl)-harnstoff (Sulfometuron-methyl),
   1-(2-Methoxycarbonylphenylsulfonyl)-3-(4-methoxy-6-methyl-1,3,5-triazin-2-yl)-3-methylharnstoff (Tribenuron-methyl),
   1-(2-Methoxycarbonylbenzylsulfonyl)-3-(4,6-dimethoxylpyrimidin-2-yl)-harnstoff (Bensulfuron-methyl),
   1-(2-Methoxycarbonylphenylsulfonyl)-3-(4,6-bis-(difluormethoxy)-pyrimidin-2-yl)-harnstoff (Primisulfuron-methyl),
   3-(4-Ethyl-6-methoxy-1,3,5-triazin-2-yl)-1-(2,3-dihydro-1,1-dioxo-2-methylbenzo[b]thiophen-7-sulfonyl)harnstoff (s. EP-A-79683),
   3-(4-Ethoxy-6-ethyl-1,3,5-triazin-2-yl)-1-(2,3-dihydro-1,1-dioxo-2-methylbenzo[b]thiophen-7-sulfonyl)harnstoff (s. EP-A-79683),
   3-(4-Methoxy-6-methyl-1,3,5-triazin-2-yl)-1-(2-methoxycarbonyl-5-iod-phenylsulfonyl)-harnstoff (s. WO 92/13845), DPX-66037, Triflusulfuron-methyl (s. Brighton Crop Prot. Conf. - Weeds-1995, S. 853),
   CGA-277476, (s, Brighton Crop Prot. Conf. - Weeds - 1995, S. 79),
   Methyl-2-[3-(4,6-dimethoxypyrimidin-2-yl)ureidosulfonyl)-4-methansulfonamido-methyl-benzoat (s. WO 95/10507),
   N,N-Dimethyl-2-[3-(4,6-dimethoxypyrimidin-2-yl)ureidosulfonyl]-4-formylaminobenzamid (s. PCT/EP 95/01344),
2) Thienylsulfonylharnstoffe, z. B.
   1-(2-Methoxycarbonylthiophen-3-yl)-3-(4-methoxy-6-methyl-1,3,5-triazin2-yl)-harnstoff (Thifensulfuron-methyl),
3) Pyrazolylsulfonylharnstoffe, z. B.
   1-(4-Ethoxycarbonyl-1-methylpyrazol-5-yl-sulfonyl)-3-(4,6-dimethoxypyrimidin-2-yl)harnstoff (Pyrazosulfuron-methyl),
   Methyl-3-chlor-5-(4,6-dimethoxypyrimidin-2-ylcarbamoylsulfamoyl)-1-methylpyrazol-4-carboxylat (s. EP 282613),
   5-(4,6-Dimethoxypyrimidin-2-yl-carbamoylsulfamoyl)-1-(2-pyridyl)-pyrazol-4-carbonsäuremethylester (NC-330, s. Brighton Crop Prot. Conference - Weeds - 1991, Vol. 1, S. 45 ff.),
   DPX-A8947, Azimsulfuron, (s. Brighton Crop Prot. Conf. - Weeds - 1995, S 65),
4) Sulfondiamid-Derivate, z.B.
   3-(4,6-Dimethoxypyrimidin-2-yl)-1-(N-methyl-N-methylsulfonylaminosulfonyl)-harnstoff (Amidosulfuron) und Strukturanaloge (s. EP-A-131258 und Z. Pfl. Krankh. Pfl. Schutz, Sonderheft XII, 489-497 (1990)),
5) Pyridylsulfonylharnstoffe, z. B.
   1-(3-N,N-Dimethylaminocarbonylpyridin-2-ylsulfonyl)-3-(4,6-dimethoxypyrimidin-2-yl)harnstoff (Nicosulfuron),
   1-(3-Ethylsulfonylpyridin-2-ylsulfonyl)-3-(-(4,6-dimethoxypyrimidin-2-yl)-harnstoff (Rimsulfuron),
   2-[3-(4,6-Dimethoxypyrimidin-2-yl)ureidosulfonyl]-6-trifluormethyl-3-pyridincarbonsäuremethylester, Natriumsalz (DPX-KE459,Flupyrsulfuron, s. Brighton Crop Prot. Conf. - Weeds - 1995, S. 49),
   Pyridylsulfonylharnstoffe, wie sie in DE-A-4000503 und DE-A-4030577 beschrieben sind, vorzugsweise solche der Formel 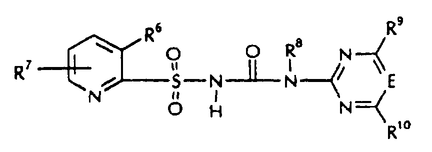 worin
      - E: CH oder N, vorzugsweise CH,
      - R⁶: lod oder NR¹¹R¹²,
      - R⁷: H, Halogen, Cyano, (C₁-C₃)-Alkyl, (C₁-C₃)-Alkoxy, (C₁-C₃)-Halogenalkyl, (C₁-C₃)-Halogenalkoxy, (C₁-C₃)-Alkylthio, (C₁-C₃)-Alkoxy-(C₁-C₃)-alkyl, (C₁-C₃)-Alkoxy-carbonyl, Mono- oder Di-((C₁-C₃)-alkyl)-amino, (C₁-C₃)-Alkyl-sulfinyl oder -sulfonyl, SO₂-NR^{a}R^{b} oder CO-NR^{a}R^{b}, insbesondere H,
      - R^{a}, R^{b}: unabhängig voneinander H, (C₁-C₃)-Alkyl, (C₁-C₃)-Alkenyl, (C₁-C₃)-Alkinyl oder zusammen -(CH₂)₄-, -(CH₂)₅- oder (CH₂)₂-O-(CH₂)₂-,
      - R⁸: H oder CH₃,
      - R⁹: Halogen, (C₁-C₂)-Alkyl, (C₁-C₂)-Alkoxy, (C₁-C₂)-Halogenalkyl, vorzugsweise CF₃, (C₁-C₂)-Halogenalkoxy, vorzugsweise OCHF₂ oder OCH₂CF₃,
      - R¹⁰: (C₁-C₂)-Alkyl, (C₁-C₂)-Halogenalkoxy, vorzugsweise OCHF₂, oder (C₁-C₂)-Alkoxy, und
      - R¹¹: (C₁-C₄)-Alkyl und
      - R¹²: (C₁-C₄)-Alkylsulfonyl oder
      - R¹¹ und R¹²: gemeinsam eine Kette der Formel -(CH₂)₃SO₂- oder-(CH₂)₄SO₂ bedeuten,
   z.B. 3-(4,6-Dimethoxypyrimid-2-yl)-1-[3-(N-methylsulfonyl-N-methylamino)pyridin-2-yl-sulfonyl]-harnstoff, oder deren Salze,
6) Alkoxyphenoxysulfonylharnstoffe, wie sie in EP-A-0342569 beschrieben sind, vorzugsweise solche der Formel 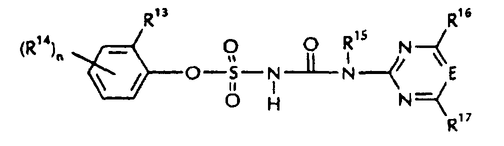 worin
   - E: CH oder N, vorzugsweise CH,
   - R¹³: Ethoxy, Propoxy oder Isopropoxy,
   - R¹⁴: Wasserstoff, Halogen, NO₂, CF₃, CN, (C₁-C₄)-Alkyl, (C₁-C₄)-Alkoxy, (C₁-C₄)-Alkylthio oder ((C₁-C₃)-Alkoxy)-carbonyl, vorzugsweise in 6-Position am Phenylring,
   - n: 1, 2 oder 3, vorzugsweise 1,
   - R¹⁵: Wasserstoff, (C₁-C₄)-Alkyl oder (C₃-C₄)-Alkenyl,
   - R¹⁶, R¹⁷: unabhängig voneinander Halogen, (C₁-C₂)-Alkyl, (C₁-C₂)-Alkoxy, (C₁-C₂)-Halogenalkyl, (C₁-C₂)-Halogenalkoxy oder (C₁-C₂)-Alkoxy-(C₁-C₂)-alkyl, vorzugsweise OCH₃ oder CH₃, bedeuten, z.B. 3-(4,6-Dimethoxypyrimidin-2-yl)-1-(2-ethoxyphenoxy)sulfonylharnstoff, oder deren Salze,
7) Imidazolylsulfonylharnstoffe, z. B.
   MON 37500, Sulfosulfuron (s. Brighton Crop Prot. Conf. - Weeds - 1995, S 57),
und andere verwandte Sulfonylharnstoffderivate und Mischungen daraus.

Geeignete Herbizide aus der Gruppe der Imidazolinone sind beispielsweise 2-(4-Alkyl-5-oxo-2-imidazolin-2-yl)-benzoesäurederivate oder 2-(4-Alkyl-5-oxo-2-imidazolin-2yl)-heteroarylcarbonsäurederivate wie z. B.
- 2-(4-Isopropyl-4-methyl-5-oxo-2-imidazolin-2-yl)-5-methylbenzoesäuremethylester und 2-(4-Isopropyl-4-methyl-5-oxo-2-imidazolin-2-yl)-4-methylbenzoesäure (Imazamethabenz),
- 5-Ethyl-2-(4-isopropyl-4-methyl-5-oxo-2-imidazolin-2-yl)-pyridin-3-carbonsäure (Imazethapyr),
- 2-(4-Isopropyl-4-methyl-5-oxo-2-imidazolin-2-yl)-chinolin-3-carbonsäure (Imazaquin),
- 2-(4-Isopropyl-4-methyl-5-oxo-2-imidazolin-2-yl)-pyridin-3-carbonsäure (Imazapyr),
- 5-Methyl-2-(4-Isopropyl-4-methyl-5-oxo-2-imidazolin-2yl)-pyridin-3-carbonsäure (Imazethamethapyr),

Geeignete Herbizide aus der Gruppe der Herbizide vom Typ der Phenoxyphenoxy- und Heteroaryloxyphenoxycarbonsäurederivate sind z. B.
a) Phenoxy-phenoxy- und Benzyloxy-phenoxy-carbonsäure-derivate, z. B.
   - 2-(4-(2,4-Dichlorphenoxy)-phenoxy)-propionsäuremethylester (Diclofopmethyl),
   - 2-(4-(4-Brom-2-chlorphenoxy)-phenoxy)-propionsäuremethylester (s. DE-A-2601548),
   - 2-(4-(4-Brom-2-fluorphenoxy)-phenoxy)-propionsäuremethylester
   - (s. US-A-4808750),
   - 2-(4-(2-Chlor-4-trifluormethylphenoxy)-phenoxy)-propionsäuremethylester (s. DE-A-2433067),
   - 2-(4-(2-Fluor-4-trifluormethylphenoxyl-phenoxy)-propionsäuremethylester (s. US-A-4808750),
   - 2-(4-(2,4-Dichlorbenzyl)-phenoxy)propionsäuremethylester (s. DE-A-2417487),
   - 4-(4-(4-Trifluormethylphenoxy)-phenoxy)-pent-2-en-säureethylester,
   - 2-(4-(4-Trifluormethylphenoxy)-phenoxy)-propionsäuremethylester (s. DE-A-2433067),
b) "Einkernige" Heteroaryloxy-phenoxy-alkancarbonsäurederivate, z. B.
   - 2-(4-(3,5-Dichlorpyridyl-2-oxy)-phenoxy)-propionsäureethylester (s. EP-A-2925),
   - 2-(4-(3,5-Dichlorpyridyl-2-oxy)-phenoxy)-propionsäurepropargylester (EP-A-3114),
   - 2-(4-(3-Chlor-5-trifluormethyl-2-pyridyloxy)-phenoxy-propionsäuremethylester (s. EP-A-3890),
   - 2-(4-(3-Chlor-5-trifluormethyl-2-pyridyloxy)-phenoxy)-propionsäureethylester (s. EP-A-3890),
   - 2-(4-(5-Chlor-3-fluor-2-pyridyloxy)-phenoxy)-propionsäurepropargylester (EP-A-191736),
   - 2-(4-(5-Trifluormethyl-2-pyridyloxy)-phenoxy)-propionsäurebutylester (Fluazifop-butyl),
c) "Zweikernige" Heteroaryloxy-phenoxy-alkancarbonsäurederivate, z. B.
   - 2-(4-<6-Chlor-2-chinoxalyloxy)-phenoxy)-propionsäure-methylester und -ethylester (Quizalofop-methyl und -ethyl),
   - 2-(4-(6-Fluor-2-chinoxalyloxy)-phenoxy)-propionsäuremethylester (s. J. Pest. Sci. Vol. 10, 61 (1985)),
   - 2-(4-(6-Chlor-2-chinoxalyloxy)-phenoxy)-propionsäure und -2-isopropylidenaminooxyethylester (Propaquizafop u. Ester),
   - 2-(4-(6-Chlorbenzoxazol-2-yl-oxy)-phenoxy)-propionsäureethylester (Fenoxaprop-ethyl), dessen D(+) Isomer (Fenoxaprop-P-ethyl) und
   - 2-(4-(6-Chlorbenzthiazol-2-yloxy)-phenoxypropionsäureethylester (s. DE-A-2640730),
   - 2-(4-(6-Chlorchinoxalyloxy)-phenoxy-propionsäure-tetrahydrofur-2-ylmethyl-ester (s. EP-A 323 727).

Geeignete Herbizide aus der Gruppe der Cyclohexandione sind beispielsweise
- 3-(1-Allyloxyiminobutyl)-4-hydroxy-6,6-dimethyl-2-oxocyclohex-3-encarbonsäuremethylester (Alloxydim),
- 2-(1-Ethoximinobutyl)-5-(2-ethylthiopropyl)-3-hydroxy-cyclohex-2-en-1-on (Sethoxydim),
- 2-(1-Ethoximinobutyl)-5-(2-phenylthiopropyl)-3-hydroxy-cyclohex-2-en-1-on (Cloproxydim),
- 2-(1-(3-Chlorallyloxy)iminobutyl)-5-[2-(ethylthio)propyl]-3-hydroxycyclohex-2-en-1-on,
- 2-(1-(3-Chlorallyloxy)iminopropyl)-5-[2-(ethylthio)propyl]-3-hydroxycyclohex-2-en-1-on (Clethodim),
- 2-(1-(Ethoxyimino)-butyl)-3-hydroxy-5-(thian-3-yl)-cyclohex-2-enon (Cycloxydim), oder
- 2-(1-Ethoxyiminopropyl)-5-(2,4,6-trimethylphenyl)-3-hydroxy-cyclohex-2-en-1-on (Tralkoxydim).

Geeignete Herbizide aus der Gruppe der Benzoylcyclohexandione sind beispielsweise
- 2-(2-Chlor-4-methylsulfonylbenzoyl)-cyclohexan-1,3-dion (SC-0051, s. EP-A-137963),
- 2-(2-Nitrobenzoyl)-4,4-dimethyl-cyclohexan-1,3-dion (s. EP-A-274634),
- 2-(2-Nitro-3-methylsulfonylbenzoyl)-4,4-dimethyl-cyclohexan-1,3-dion (s. WO 91/13548).

Geeignete Herbizide aus der Gruppe der Pyrimidinyloxy-pyrimidincarbonsäure- bzw. Pyrimidinyloxy-benzoesäure-Derivate sind beispielsweise
- 3-(4,6-Dimethoxypyrimidin-2-yl)-oxy-pyridin-2-carbonsäurebenzylester (EP-A-249 707),
- 3-(4,6-Dimethoxypyrimidin-2-yl)-oxy-pyridin-2-carbonsäuremethylester (EP-A-249 707),
- 2,6-Bis[(4,6-dimethoxypyrimidin-2-yl)-oxy]-benzoesäure (EP-A-321 846),
- 2,6-Bis[(4,6-dimethoxypyrimidin-2-yl)-oxy]-benzoesäure-(1-ethoxycarbonyloxyethyl)-ester (EP-A-472 113).

Geeignete Herbizide aus der Gruppe der Triazolopyrimidinsulfonamide sind beispielsweise
- N-(2,6-Difluorphenyl)-7-methyl-1,2,4-triazolo-(1,5-c)-pyrimidin-2-sulfonamid (Flumetsulam),
- N-(2,6-Dichlor-3-methylphenyl)-5,7-dimethoxy-1,2,4-triazolo-(1,5-c)-pyrimidin-2-sulfonamid,
- N-(2,6-Difluorphenyl)-7-fluor-5-methoxy-1,2,4-triazolo-(1,5-c)-pyrimidin-2-sulfonamid
- N-(2,6-Dichlor-3-methylphenyl)-7-chlor-5-methoxy-1,2,4-triazolo-(1,5-c)-pyrimidin-2- sulfonamid,
- N-(2-Chlor-6-methoxycarbonyl)-5,7-dimethyl-1,2,4-triazolo-(1,5-c)-pyrimidin-2-sulfonamid (siehe z. B. EP-A-343 752, US-A-4 988 812).

Ein geeignetes Herbizid aus der Gruppe der S-(N-Aryl-N-alkyl-carbamoylmethyl)dithiophosphorsäureester ist beispielsweise
- S-[N-(4-Chlorphenyl)-N-isopropyl-carbamoylmethyl]-O,O-dimethyldithiophosphat (Anilofos).

Die obengenannten Herbizide sind dem Fachmann bekannt und in der Regel in "The Pesticide Manual",The British Crop Protection Council and the Royal Soc. of Chemistry, 10th edition, 1994 oder in "Agricultural Chemicals Book II - Herbicides -", by W.T. Thompson, Thompson Publications, Fresno CA, USA 1990 oder in "Farm Chemicals Handbook '90", Meister Publishing Company, Willoughby OH, USA 1990 beschrieben.

Die herbizide Wirkstoffe und die erwähnten Safener können zusammen (als fertige Formulierung oder im Tank-mix-Verfahren) oder in beliebiger Reihenfolge nacheinander ausgebracht werden. Das Gewichtsverhältnis Safener:Herbizid kann innerhalb weiter Grenzen variieren und liegt vorzugsweise im Bereich von 1:10 bis 10:1, insbesondere von 1:10 bis 5:1. Die jeweils optimalen Mengen an Herbizid und Safener sind vom Typ des verwendeten Herbizids oder vom verwendeten Safener sowie von der Art des zu behandelnden Pflanzenbestandes abhängig und lassen sich von Fall zu Fall durch entsprechende Vorversuche ermitteln.

Haupteinsatzgebiete für die Anwendung der Safener sind vor allem Getreidekulturen (Weizen, Roggen, Gerste, Hafer), Reis, Mais, Sorghum, aber auch Baumwolle und Sojabohne, vorzugsweise Getreide und Mais.

Ein besonderer Vorteil der erfindungsgemäßen Safener der Formel (I) ist bei deren Kombination mit Herbiziden aus der Gruppe der Sulfonylharnstoffe festzustellen. Einige Herbizide dieser Strukturklasse können speziell in Getreidekulturen und/oder Mais nicht oder nicht genügend selektiv eingesetzt werden. Durch die Kombination mit den erfindungsgemäßen Safenern sind auch bei diesen Herbiziden in Getreide oder Mais hervorragende Selektivitäten zu erreichen.

Die Safener der Formel (I) können je nach ihren Eigenschaften zur Vorbehandlung des Saatgutes der Kulturpflanze (Beizung der Samen) verwendet werden oder vor der Saat in die Saatfurchen eingebracht oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandllung der angesäten, aber noch nicht bewachsenen Anbauflächen ein. Bevorzugt ist die gemeinsame Anwendung mit dem Herbizid. Hierzu können Tankmischungen oder Fertigformulierungen eingesetzt werden.

Die benötigten Aufwandmengen der Safener können je nach Indikation und verwendetem Herbizid innerhalb weiter Grenzen schwanken und liegen in der Regel im Bereich von 0,001 bis 5 kg/ha, vorzugsweise 0,005 bis 0,5 kg/ha, insbesondere 5-100 g/ha Aktivsubstanz.

Gegenstand der vorliegenden Erfindung ist deshalb auch ein Verfahren zum Schutz von Kulturpflanzen vor phytotoxischen Nebenwirkungen von Herbiziden aus der Gruppe der Sulfonylharnstoffe, das dadurch gekennzeichnet ist, daß eine wirksame Menge einer Verbindung der Formel (I) vor, nach oder gleichzeitig mit dem Herbizid auf die Pflanzen, Pflanzensamen oder die Anbaufläche appliziert wird.

Gegenstand der Erfindung sind auch pflanzenschützende Mittel, die einen Wirkstoff der Formel (I) und übliche Formulierungshilfsmittel enthalten, sowie herbizide Mittel, die einen Wirkstoff der Formel I und ein Herbizid aus der Gruppe der Sulfonylharnstoffe sowie im Bereich des Pflanzenschutzes übliche Formulierungshilfsmittel enthalten.

Die Verbindungen der Formel (I) und deren Kombinationen mit einem oder mehreren der genannten Herbizide können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖI-Emulsionen, versprühbare Lösungen oder Suspensionen, Suspoemulsionen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, Granulate für die Boden- bzw Streuapplikation, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie",Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden:
Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.
Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.
Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.
Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I) (Safener) oder des Safener/Herbizid-Wirkstoffgemischs und 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Zur Anwendung werden die in handelsüblicher Form vorliegenden Formulierungen gegebenenfalls in üblicher Weiswe verdünnt, z.B. bei Spritzpulvern, emulgierbaren Konzentraten, Dispersionen und wasserdispergierbaren Granulaten mittels Wasser. Staubförmige Zubereitungen, Granulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids u. a. variiert die erforderliche Aufwandmenge der Safener.

### A. Formulierungsbeispiele

a) Ein Stäubemittel wird erhalten, indem man 10 Gew.-Teile einer Verbindung der Formel (I) oder eines Wirkstoffgemischs aus einem Herbizid und einem Safener der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) oder eines Wirkstoffgemischs aus einem Herbizid und einem Safener der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) oder eines Wirkstoffgemischs aus einem Herbizid und einem Safener der Formel (I) mit 6 Gew.-Teilen Alkylphenolpolyglykolether (® Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel I oder eines Wirkstoffgemischs aus einem Herbizid und einem Safener der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| | | |
|---|---|---|
| 75 | Gewichtsteile | einer Verbindung der Formel (I) oder eines Wirkstoffgemischs aus einem Herbizid und einem Safener der Formel (I), |
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man

| | | |
|---|---|---|
| 25 | Gewichtsteile | einer Verbindung der Formel (I) oder eines Wirkstoffgemischs aus einem Herbizid und einem Safener der Formel (I), |
| 5 | " | 2,2'-dinaphthylmethan-6,6'-disulfonsaures Natrium |
| 2 | " | oleoylmethyltaurinsaures Natrium, |
| 1 | Gewichtsteil | Polyvinylalkohol, |
| 17 | Gewichtsteile | Calciumcarbonat und |
| 50 | " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### B. Herstellungsbeispiele

### 1. N-[4-(2-Methoxybenzoylsulfamoyl)phenyl]-cyclopentancarboxamid (Beispiel 172 aus Tabelle 1)

### 1a. 2-Methoxy-N-(4-nitrophenylsulfonyl)-benzamid

30,0 g (0,15 mol) 4-Nitrobenzolsulfonamid werden mit 30,0 g (0,3 mol) Triethylamin in 300 ml Acetonitril versetzt und bei 50 °C mit 27,8 g (0,16 mol) o-Anisoylchlorid - in 20 ml Acetonitril gelöst - versetzt. Nach 3 h bei 50 °C wird in 80 ml Eiswasser eingerührt und der Niederschlag abgesaugt und getrocknet. Weiteres Produkt kann durch Ansäuern der Mutterlauge erhalten werden; Ausbeute: 41,4 g (83 %); Schmp.: 154 - 158 °C.

### 1b. 2-Methoxy-N-(4-aminophenylsulfonyl)-benzamid

50,0 g (0,15 mol) 2-Methoxy-N-(4-nitrophenylsulfonyl)-benzamid werden in einer Mischung von 450 ml Ethanol und 750 ml 2n HCI suspendiert und auf 50°C erwärmt. Bei dieser Temperatur werden 97,2 g (1,5mol) Zinkpulver portionsweise zugegeben und weitere 2 h gerührt. Nach Abkühlung wird filtriert, das Filtrat bis zur Hälfte eingeengt und gekühlt, der Niederschlag abgesaugt und getrocknet; Ausbeute: 43,6 g (96 %); Schmp.: 180 - 182 °C.

### 1c. N-[4-(2-Methoxybenzoylsulfamoyl)phenyl]-cyclopentancarboxamid

2,8 g (9 mmol) 2-Methoxy-N-(4-aminophenylsulfonyl)-benzamid werden in 100 ml Dioxan suspendiert, bei 0 °C mit 0,72 g (9 mmol) Pyridin und 1,21 g (9 mmol) Cyclopentancarbonsäurechlorid versetzt und 2 h bei dieser Temperatur gerührt. Anschließend giebt man die Reaktionsmischung in Wasser und saugt den erhaltenen Niederschlag ab. Nach Trocknung werden 2,68 g (73 %) N-[4-(2-Methoxybenzoylsulfamoyl)phenyl]-cyclopentancarboxamid mit einem Schmelzpunkt von 202 - 206 °C erhalten.

### 2. N-[4-(2-Methoxybenzoylsulfamoyl)phenyl]-2,4-dichlorbenzamid (Beispiel 215 aus Tabelle 1)

### 2a. N-(4-Sulfamoylphenyl)-2,4-dichlorbenzamid

10 g (60mmol) Sulfanilamid werden in 150ml Dioxan suspendiert und mit 4,6 g (60 mmol) Pyridin versetzt. Bei 0 °C werden dann 12,2 g (60 mmol) 2,4-Dichlorbenzoylchlorid zugegeben und weitere 2h bei Raumtemperatur nachgerührt. Die Mischung wird in 200 ml Wasser eingerührt, der ausfallende Niederschlag wird abgesaugt und getrocknet; Ausbeute: 16,9 g (85 %); Schmp.: 228 - 232 °C.

### 2b. N-[4-(2-Methoxybenzoylsulfamoyl)phenyl]-2,4-dichlorbenzamid

5 g (15 mmol) N-(4-Sulfamoylphenyl)-2,4-dichlorbenzamid werden bei 0 °C in 80 ml Acetonitril vorgelegt und mit 2,93 g (30mmol) Triethylamin sowie einer katalytischen Menge DMAP (4-Dimethylaminopyridin) versetzt. Anschließend tropft man 2,47 g (15 mmol) o-Anisoylchlorid - in 20 ml Acetonitril gelöst - hinzu. Nach weiteren 2 h bei Raumtemperatur wird in Wasser gegeben und der Niederschlag abgesaugt; Ausbeute: 4,2g (60 %); Schmp.: 140 -146 °C.

### 3. 2-Methoxy-N-[4-(2-methoxybenzoylsulfamoyl)phenyl]-acetamid (Beispiel 160 aus Tabelle 1}

### 3a. 2-Methoxy-N-(4-sulfamoylphenyl)-acetamid

10 g (0,06 mol) Sulfanilamid werden in 150ml Dioxan suspendiert und mit 4,6 g (60 mmol) Pyridin versetzt. Bei 0 °C werden dann 6,3 g (60 mmol) Methoxyessigsäurechlorid zugegeben und weitere 2h bei Raumtemperatur nachgerührt. Die Mischung wird in 200 ml Wasser eingerührt, der ausfallende Niederschlag wird abgesaugt und getrocknet; Ausbeute: 12,9 g (91 %); Schmp.: 200 - 208 °C.

### 3b. 2-Methoxy-N-[4-(2-methoxybenzoylsulfamoyl)phenyl]-acetamid

8 g (33 mmol) 2-Methoxy-N-(4-sulfamoylphenyl)-acetamid werden in 160 ml Acetonitril bei 0°C vorgelegt und mit 6,63 g (66 mmol) Triethylamin sowie einer katalytischen Menge DMAP versetzt. Anschließend gibt man 5,6 g (33 mmol) 2-Methoxybenzoylchlorid - in 20 ml Acetonitril gelöst - bei 0 °C hinzu und rührt weitere 2 h bei dieser Temperatur. Nach weiteren 2 h bei Raumtemperatur wird in Wasser gegeben und der Niederschlag abgesaugt;
Ausbeute: 10,5 g (84 %); Schmp.: 170-172 °C.

In den nachfolgenden Tabellen sind beispielhaft eine Reihe von Verbindungen der allgemeinen Formel (I) aufgeführt, die in analoger Weise zu den obigen Beispielen 1 bis 3 und den weiter oben erwähnten Methoden erhalten werden können. In den Tabellen bedeuten:
Me = Methyl
Et = Ethyl
n-Pr = n-Propyl
i-Pr = Isopropyl
c-Pr = Cyclopropyl
n-, i-, t- oder s-Bu = normal-(geradkettiges), iso-, tertiär- oder sekundär-Butyl
c-Bu = Cyclobutyl
Schmp. = Schmelzpunkt (in °C)

Indexzahlen sind aus Platzgründen nicht tiefgestellt bzw. hochgestellt; so bedeutet z.B. OCF3 den Rest Trifluormethoxy = OCF₃, usw. In der Kopfzeile bedeutet R1 = R¹, usw.

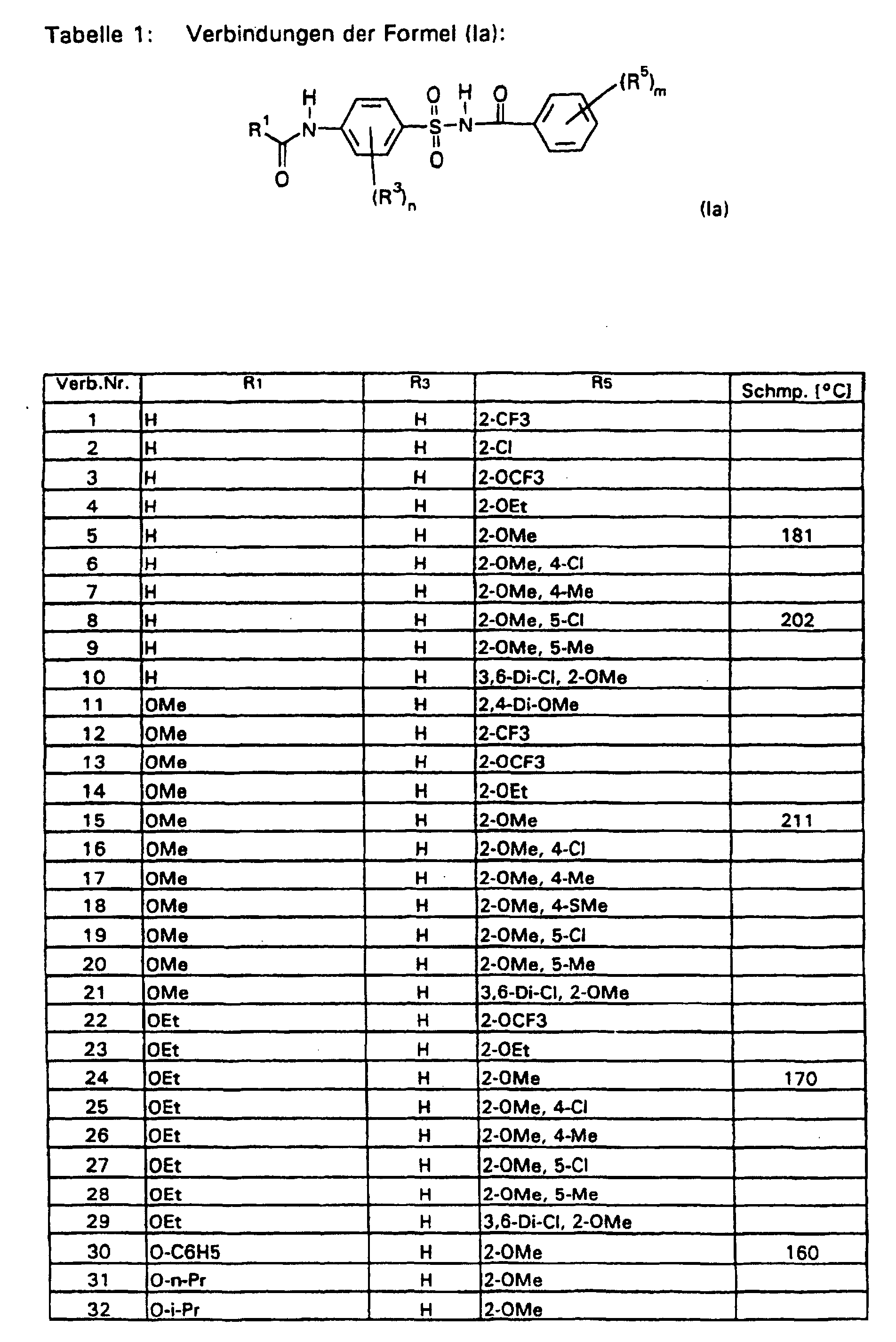

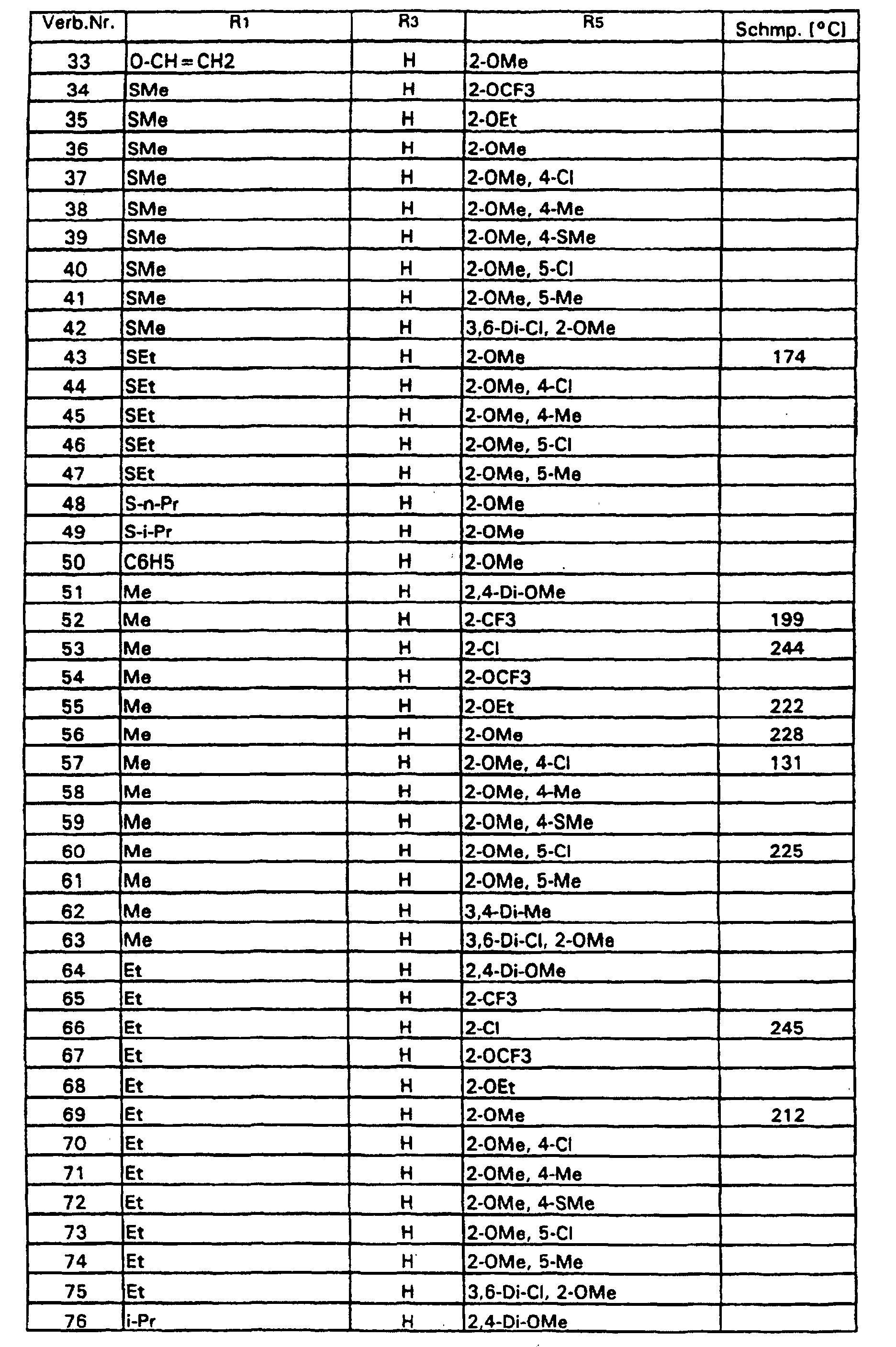

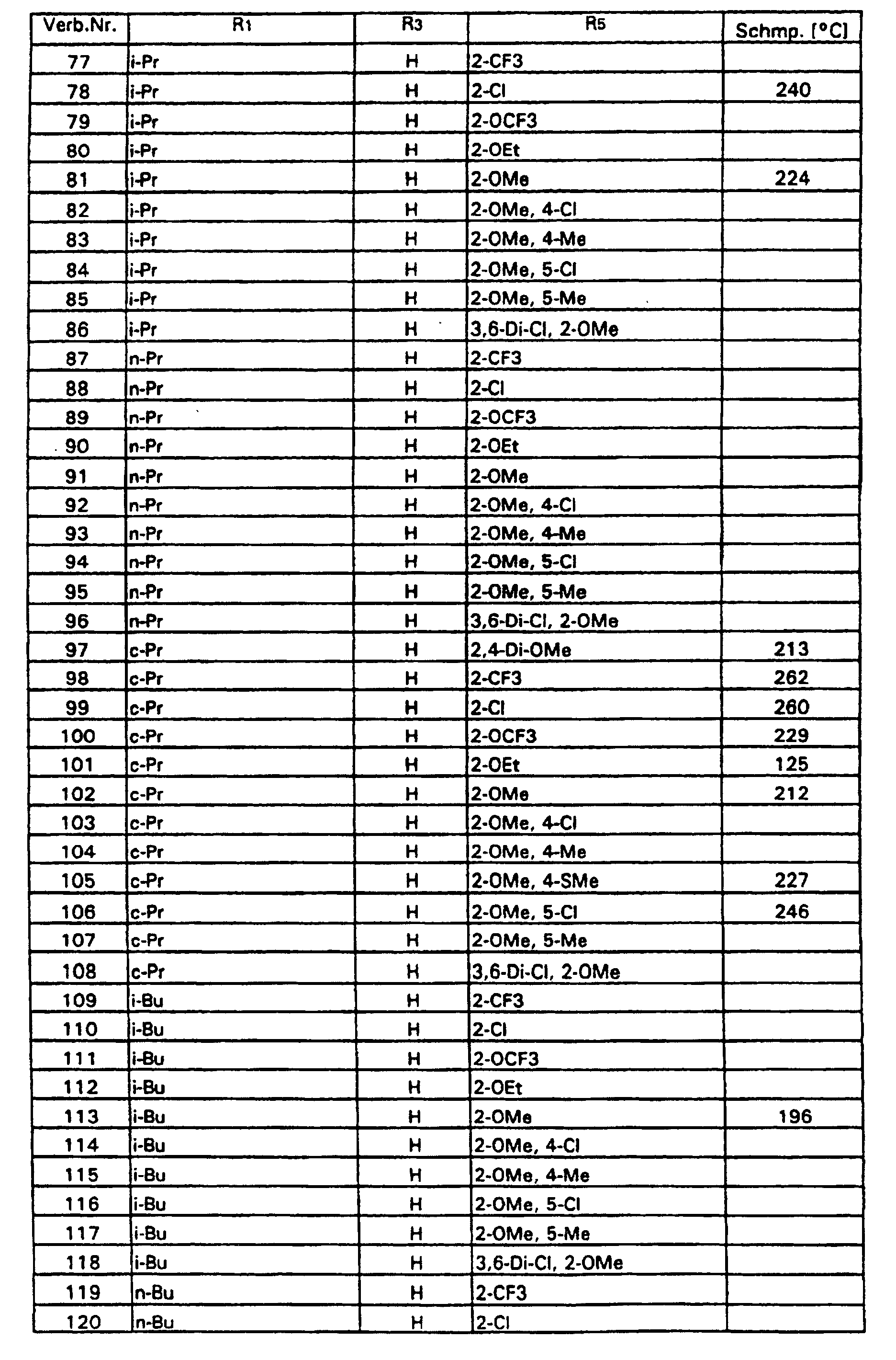

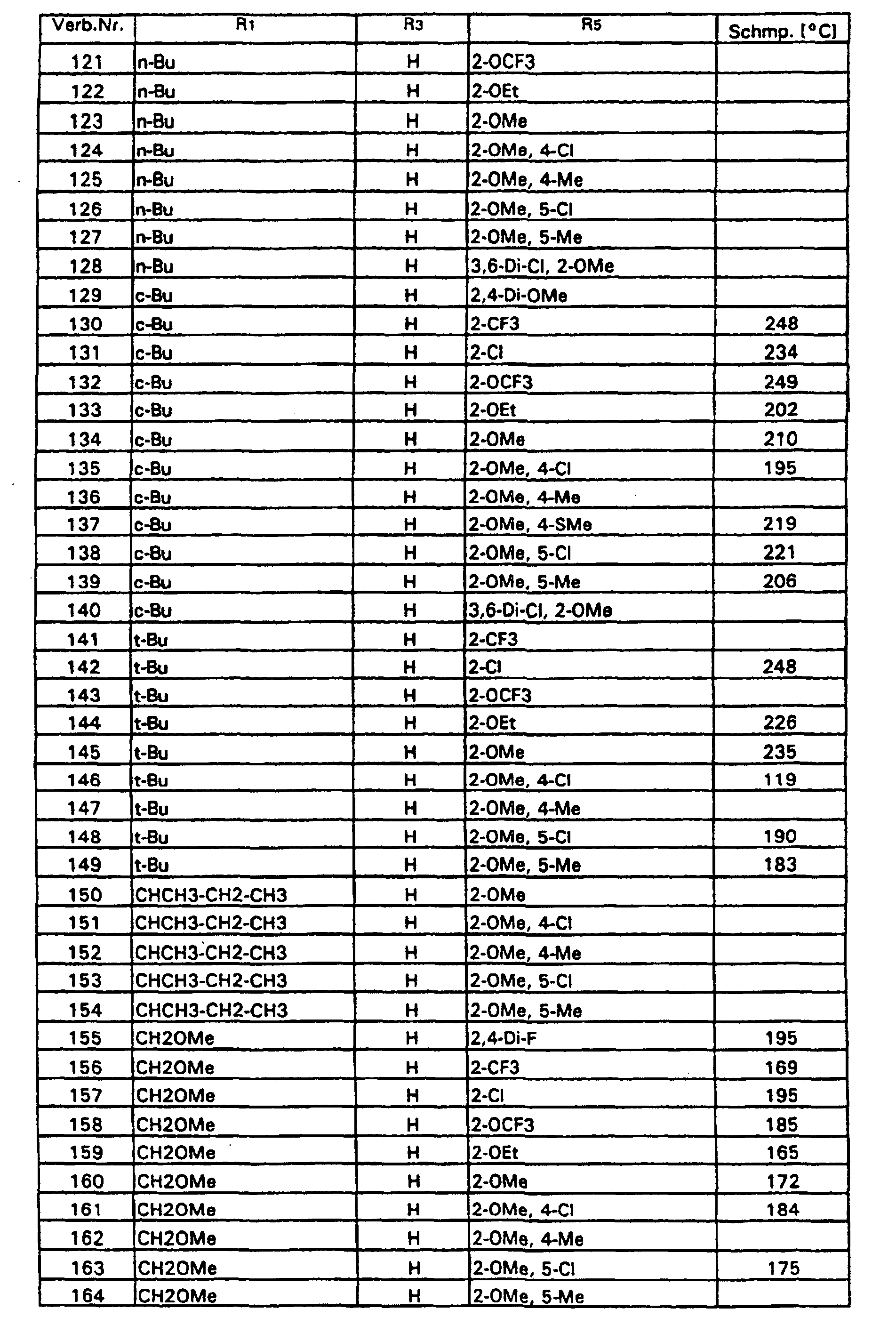

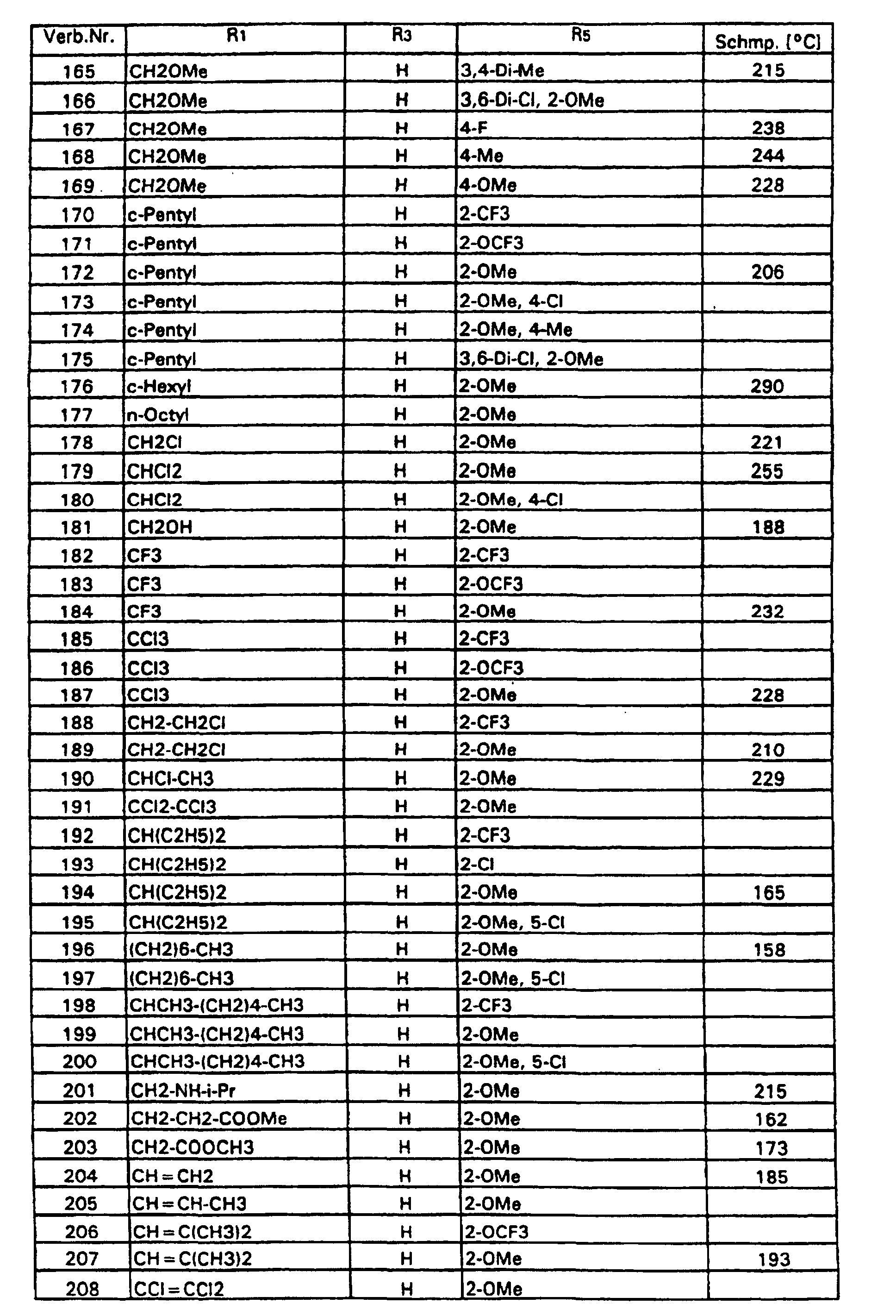

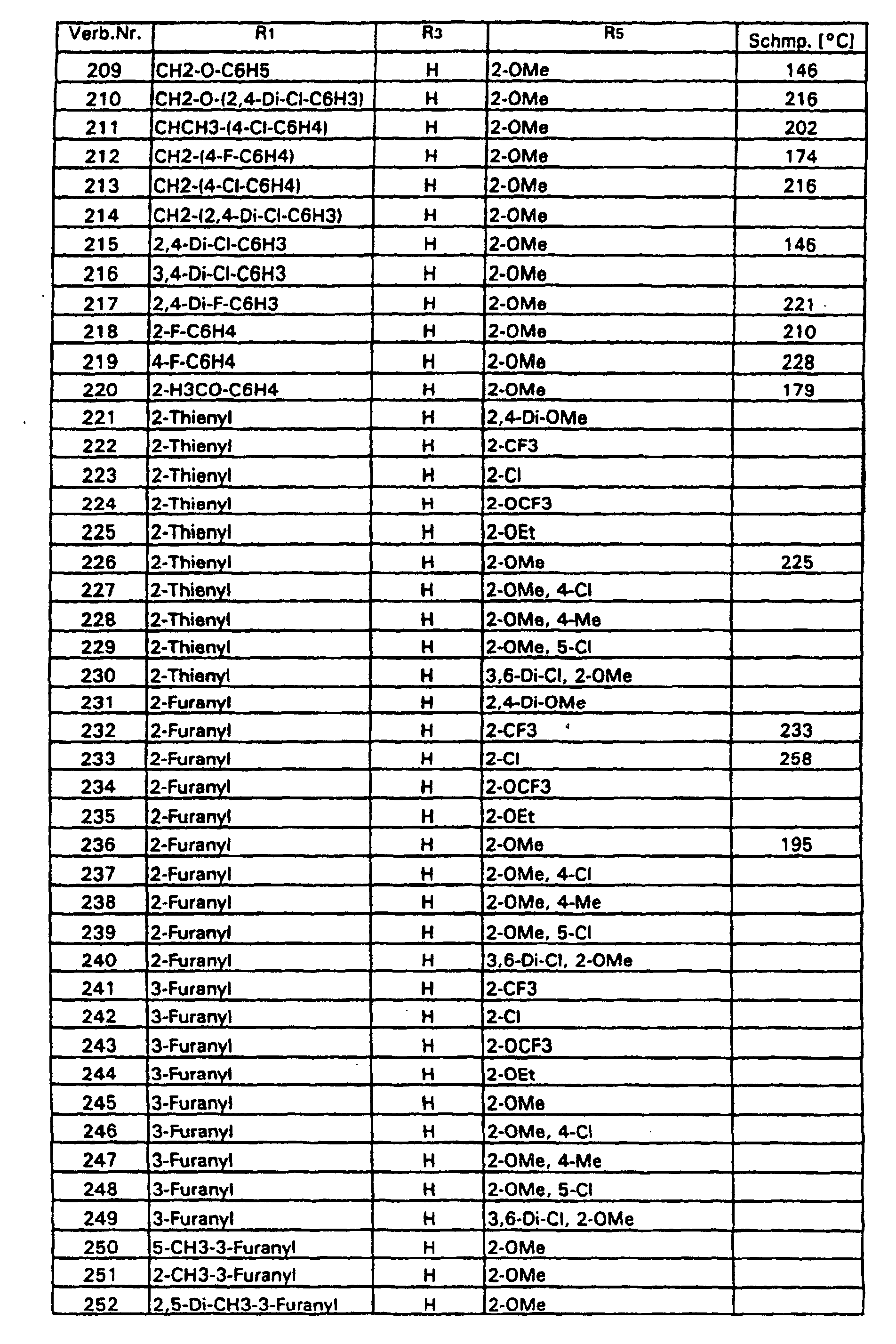

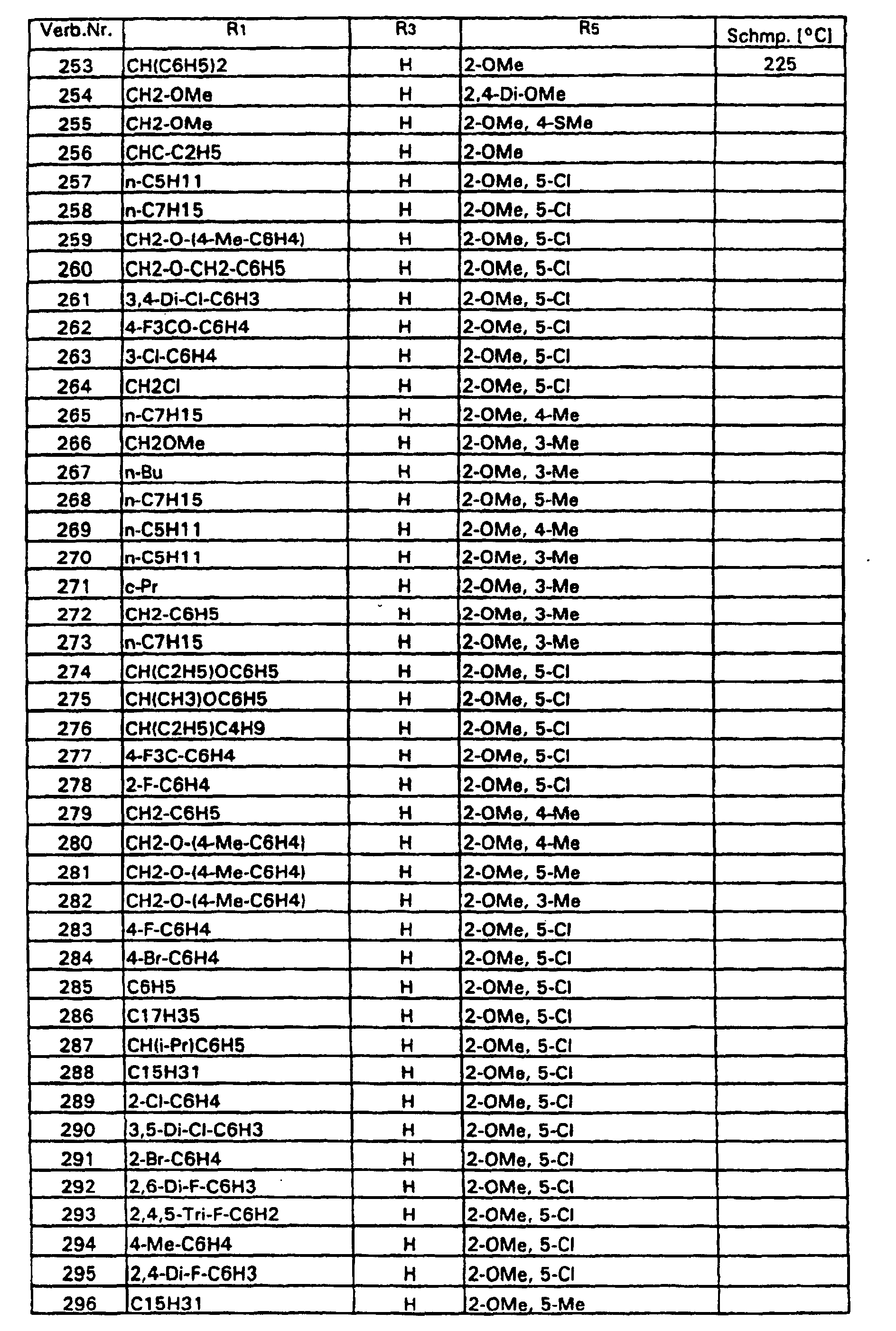

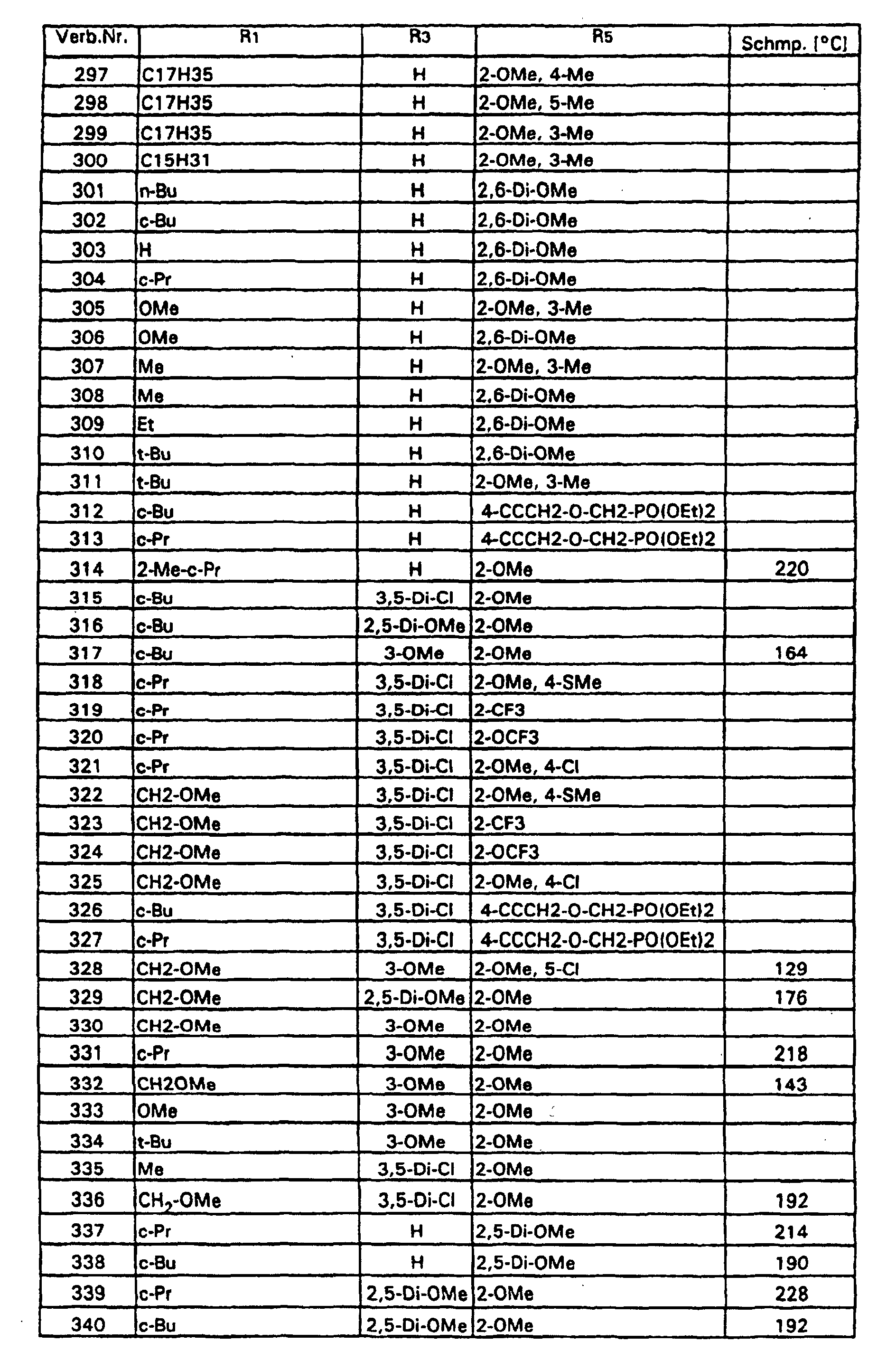

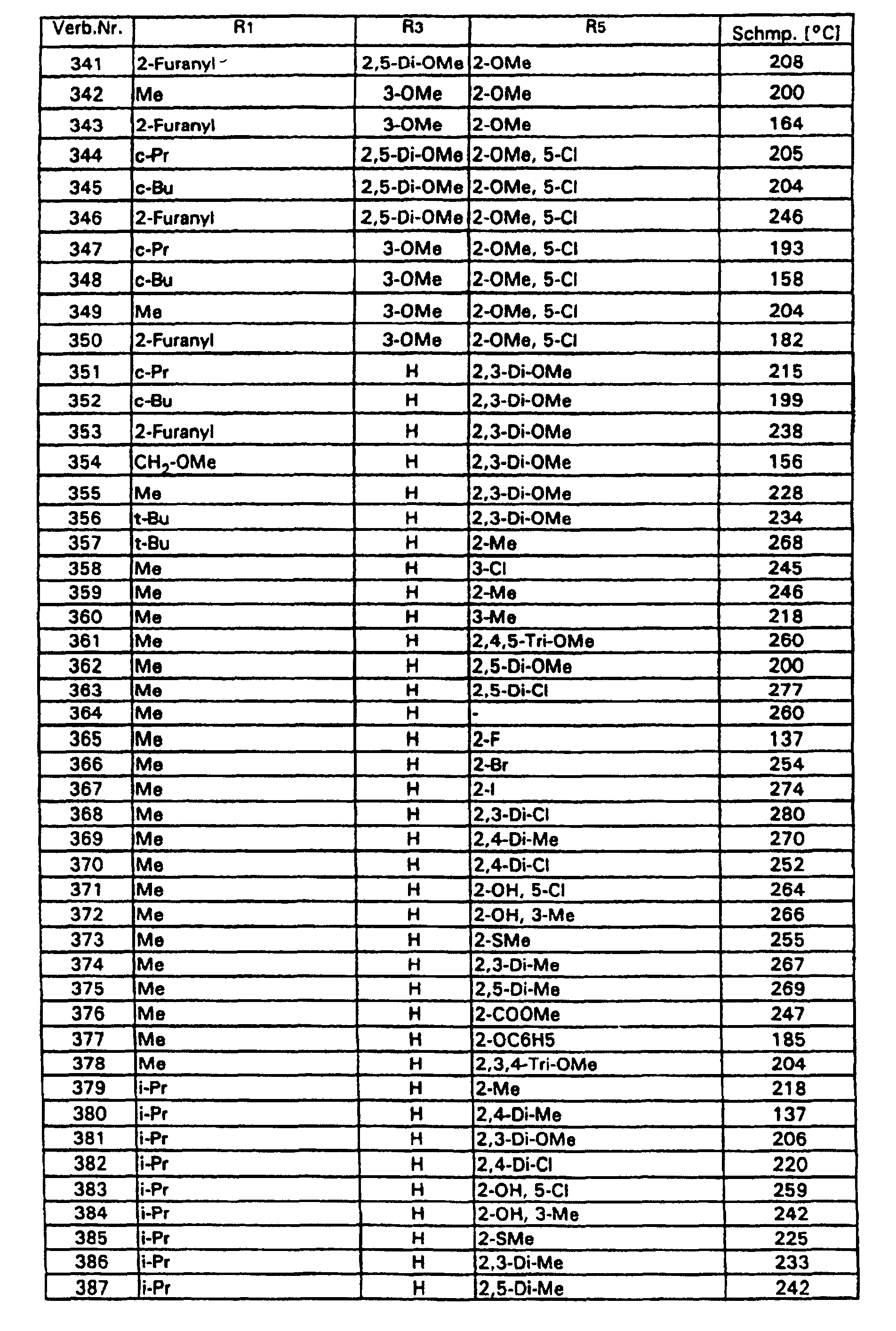

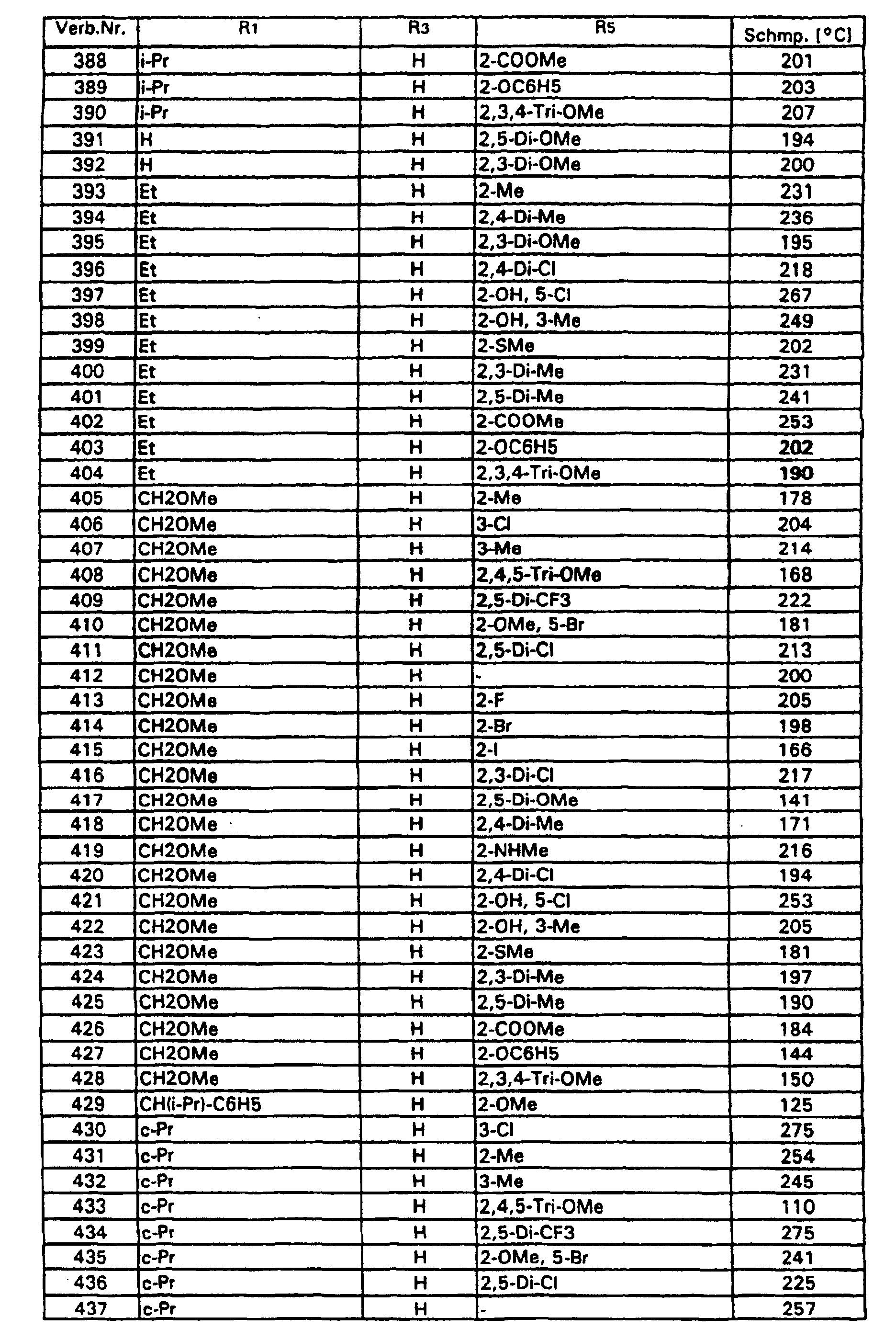

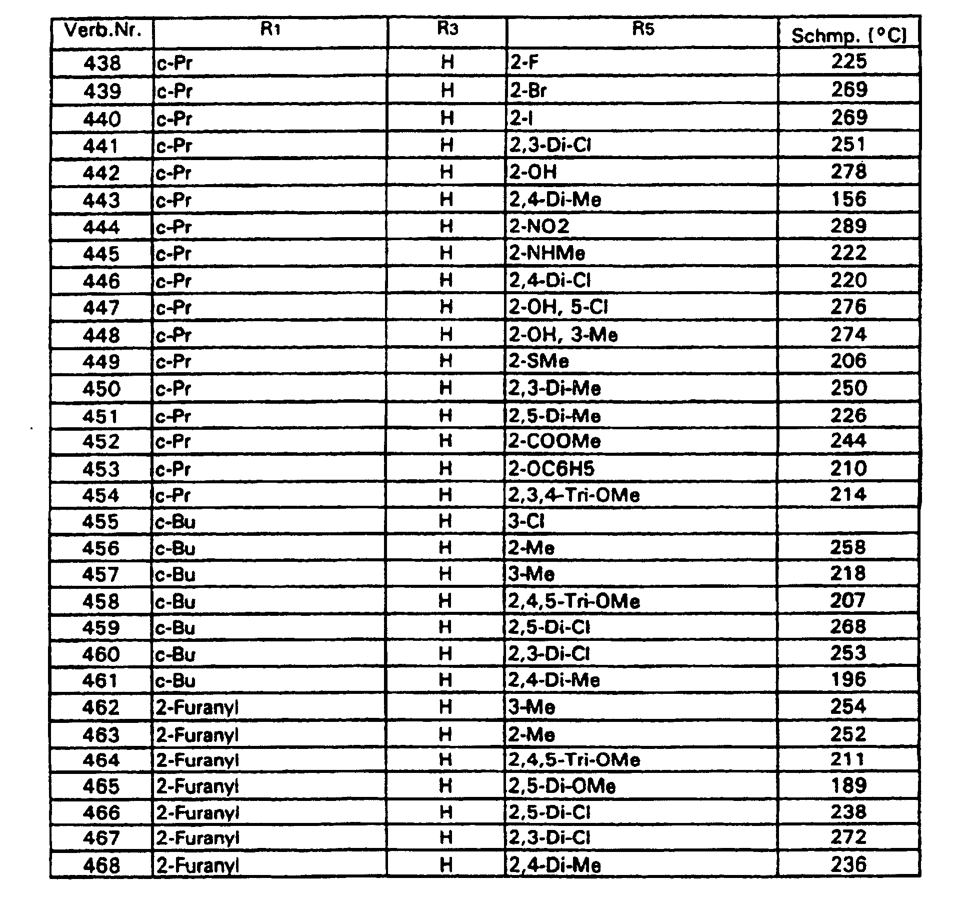

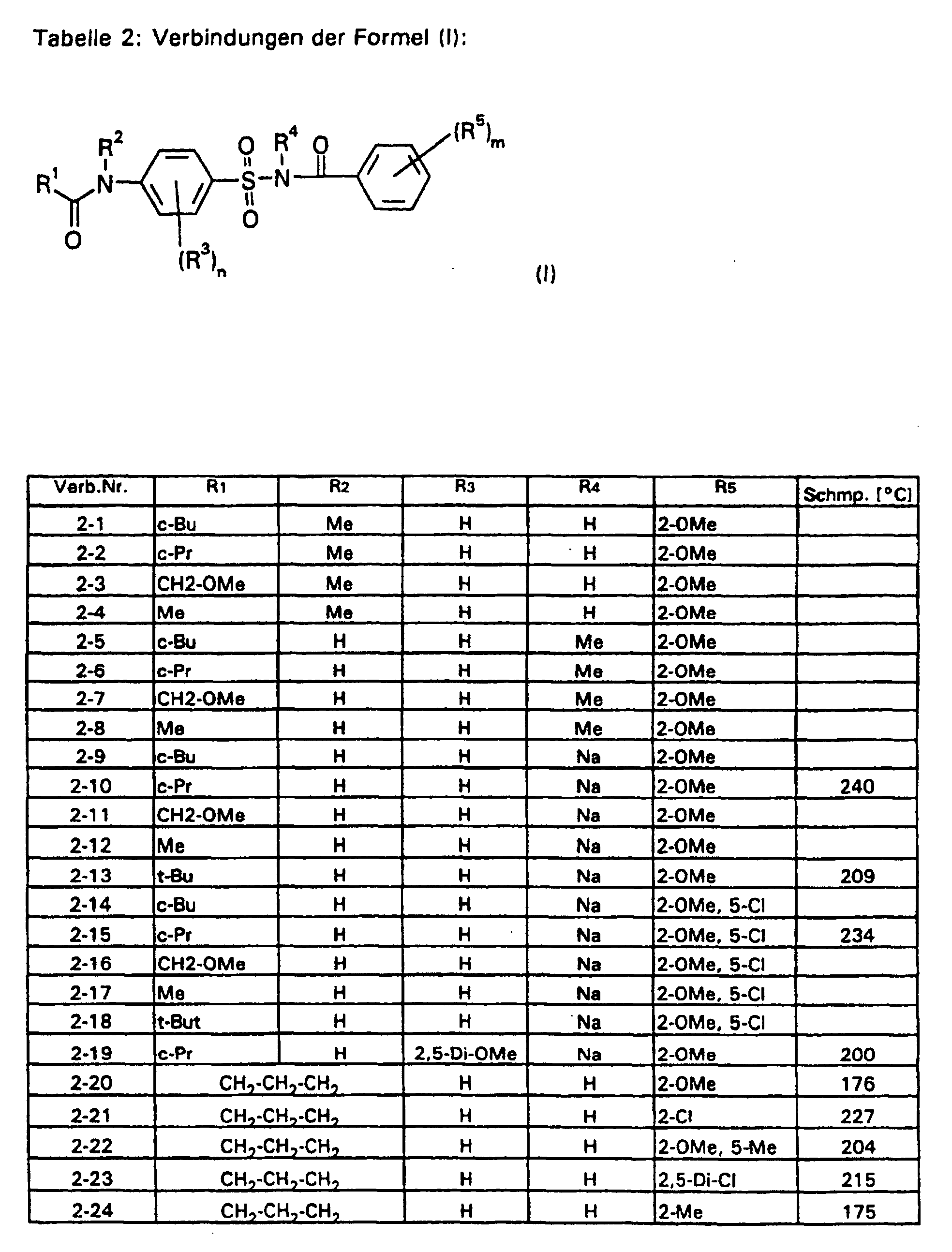

### C. Biologische Beispiele

### 1. Bonitierung der Schadwirkung

Die Schadwirkung an den Pflanzen wird nach einer Skala von 0-100% optisch im Vergleich zu Kontrollpflanzen bewertet:
0% = keine erkennbare Wirkung im Vergleich zur unbehandelten Pflanze,
100% = behandelte Pflanze stirbt ab.

### 2. Herbizidwirkung und Safenerwirkung im Vorauflauf

Samen bzw. Rhizomstücke von mono- und dikotylen Unkrautpflanzen sowie von Kulturpflanzen werden in Plastiktöpfen von 9 cm Durchmesser in sandiger Lehmerde ausgelegt und mit Erde abgedeckt. Alternativ werden für den Test unter Bedingungen für Paddy-Reis im Reisanbau vorkommende Unkräuter im mit Wasser gesättigten Boden kultiviert, wobei so viel Wasser in die Töpfe gefüllt wird, daß das Wasser bis zu Bodenoberfläche oder einige Millimeter darüber steht. Die in Form von Emulsionskonzentraten formulierten erfindungsgemäßen Herbizid-Safener-Wirkstoffkombinationen sowie in parallelen Versuchen die entsprechend formulierten Einzelwirkstoffe werden dann als Emulsionen mit einer Wasseraufwandmenge von umgerechnet 300 I/ha, in unterschiedlichen Dosierungen auf die Oberfläche der Abdeckerde appliziert oder beim Reis ins Bewässerungswasser gegossen.

Nach der Behandlung werden die Töpfe im Gewächshaus aufgestellt und unter guten Wachstumsbedingungen für die Unkräuter gehalten. Die optische Bonitur der Pflanzen- bzw. der Auflaufschäden erfolgt nach dem Auflaufen der Versuchspflanzen nach einer Versuchszeit von 2 Wochen im Vergleich zu unbehandelten Kontrollen. Wie die Versuche zeigen, weisen die erfindungsgemäßen herbiziden Mittel, welche z. B. einen Safener der Beispiele 5, 15, 30, 43, 56, 60, 81, 97, 98, 99, 101, 102, 103, 106, 134, 145, 155, 156, 157, 158, 160, 179, 181, 189, 194, 204, 207, 219, 236, 245, 363, 401, 405, 425, 431, 436, 465, 2-10, 2-15 und 2-20 in Kombination mit dem Sulfonylharnstoffherbizid 3-(4,6-Dimethoxypyrimid-2-yl)-1-[3-(N-methylsulfonyl-N-methylaminol-pyridyl-2-yl-sulfonyl]-harnstoff oder 3-(4-Methoxy-6-methyl-1,3,5-triazin-2-yl)-1-(2-methoxycarbonyl-5-iod-phenylsulfonyl)-harnstoff (Natriumsalz) oder 1-(2-Methoxycarbonylthiophen-3-yl)-3-(4-methoxy-6-methyl-1,3,5-triazin-2-yl)harnstoff (Thifensulfuron-methyl) oder 1-(3-N,N-Dimethylaminocarbonylpyridin-2-ylsulfonyl)-3-(4,6-dimethoxypyrimidin-2-yl)harnstoff (Nicosulfuron) oder 1-(3-Ethylsulfonylpyridin-2-ylsulfonyl)-3-(4,6-dimethoxypyrimidin-2-yl)harnstoff (Rimsulfuron) oder N,N-Dimethyl-2-[3-(4,6-dimethoxypyrimidin-2-yl)ureidosulfonyl]-4-formylaminobenzamid oder Methyl-2-[3-(4,6-dimethoxypyrimidin-2-yl)ureidosulfonyl]-4-methansulfonamidomethylbenzoat oder mit dem Imidazolinonherbizid 5-Ethyl-2-(4-isopropyl-4-methyl-5-oxo-2-imidazolin-2-yl)pyridin-3-carbonsäure (Imazethapyr) oder mit dem Aryloxyphenoxyherbizid 2-(4-(6-Chlorbenzoxazol-2-yloxy)-phenoxy)-propionsäureethylester (Fenoxapropethyl) oder mit 2,6-Bis[(4,6-dimethoxypyrimidin-2-yl)oxy]-benzoesäure Natriumsalz im Verhältnis von Herbizid:Safener von 2:1 bis 1:20 enthalten, eine gute herbizide Vorauflaufwirksamkeit gegen ein breites Spektrum von Ungräsern und Unkräutern auf, wobei Schäden an Kulturpflanzen wie Mais, Reis, Weizen oder Gerste oder anderem Getreide im Vergleich zur Anwendung der einzelnen Herbizide ohne Safener wesentlich reduziert sind, d.h. um 30% bis zu 100% weniger Herbizidschäden aufweisen.

### 3. Herbizidwirkung und Safenerwirkung im Nachauflauf

Samen bzw. Rhizomstücke von mono- und dikotylen Unkräutern und von Kulturpflanzen werden in Plastiktöpfen in sandigem Lehmboden ausgelegt, mit Erde abgedeckt und im Gewächshaus unter guten Wachstumsbedingungen angezogen. Alternativ werden für den Test unter Bedingungen für Paddy-Reis im Reisanbau vorkommende Unkräuter in Töpfen angezogen, in denen Wasser bis zu 2 cm über der Bodenoberfläche steht, und während der Versuchsphase kultiviert. Drei Wochen nach der Aussaat werden die Versuchspflanzen im Dreiblattstadium behandelt. Die als Emulsionskonzentrate formulierten erfindungsgemäßen Herbizid-Safener-Wirkstoffkombinationen sowie in parallelen Versuchen die entsprechend formulierten Einzelwirkstoffe werden in verschiedenen Dosierungen mit einer Wasseraufwandmenge von umgerechnet 300 I/ha auf die grünen Pflanzenteile gesprüht und nach 2 Wochen Standzeit der Versuchspflanzen im Gewächshaus unter optimalen Wachstumsbedingungen die Wirkung der Präparate optisch im Vergleich zu unbehandelten Kontrollen bonitiert. Bei Reis oder bei Unkräutern, die im Reisanbau vorkommen, werden die Wirkstoffe auch direkt ins Bewässerungswasser gegeben (Applikation in Analogie zur sogenannten Granulatanwendung) oder auf Pflanzen und ins Bewässerungswasser gesprüht. Wie die Versuche zeigen, weisen die erfindungsgemäßen herbiziden Mittel, welche z. B. einen Safener der Beispiele 5, 15, 30, 43, 56, 60, 81, 97, 98, 99, 101, 102, 103, 106, 134, 145, 155, 156, 157, 158, 160, 179, 181, 189, 194, 204, 207, 219, 236, 245, 363, 401, 405, 425, 431, 436, 465, 2-10, 2-15 und 2-20 in Kombination mit dem Sulfonylharnstoffherbizid 3-(4,6-Dimethoxypyrimid-2-yl)-1-[3-(N-methylsulfonyl-N-methylamino)pyridin-2-yl-sulfonyl]-harnstoff oder 3-(4-Methoxy-6-methyl-1,3,5-triazin-2-yl)-1-(2-methoxycarbonyl-5-iod-phenylsulfonyl)-harnstoff (Natriumsalz) oder 1-(2-Methoxycarbonylthiophen-3-yl)-3-(4-methoxy-6-methyl-1,3,5-triazin-2-yl)harnstoff (Thifensulfuron-methyl) oder 1-(3-N,N-Dimethylaminocarbonylpyridin-2-ylsulfonyl)-3-(4,6-dimethoxypyrimidin-2-yl)harnstoff (Nicosulfuron) oder 1-(3-Ethylsulfonylpyridin-2-ylsulfonyl)-3-(4,6-dimethoxypyrimidin-2-yl)harnstoff (Rimsulfuron) oder N,N-Dimethyl-2-[3-(4,6-dimethoxypyrimidin-2-yl)ureidosulfonyl]-4-formylaminobenzamid oder Methyl-2-[3-(4,6-dimethoxypyrimidin-2-yl)ureidosulfonyl]-4-methansulfonamidomethylbenzoat oder mit dem Imidazolinonherbizid 5-Ethyl-2-(4-isopropyl-4-methyl-5-oxo-2-imidazolin-2-yl)-pyridin-3-carbonsäure (Imazethapyr) oder mit dem Aryloxyphenoxyherbizid 2-(4-(6-Chlorbenzoxazol-2-yloxy)-phenoxy)-propionsäureethylester (Fenoxaprop-ethyl) oder mit 2,6-Bis[(4,6-dimethoxypyrimidin-2-yl)oxy]-benzoesäure Natriumsalz im Verhältnis von Herbizid:Safener von 2:1 bis 1:20 enthalten, auch im Nachauflauf eine gute herbizide Wirksamkeit gegen ein breites Spektrum von Ungräsern und Unkräutern auf, wobei Schäden an Kulturpflanzen wie Mais, Reis, Weizen oder Gerste im Vergleich zur Anwendung der einzelnen Herbizide ohne Safener wesentlich reduziert sind, d.h. um 30% bis zu 100% weniger Herbizidschäden aufweisen.

## Patentansprüche

1. Verwendung von Verbindungen der Formel (I) oder deren Salze als Safener zum Schützen von Kulturpflanzen vor phytotoxischen Nebenwirkungen von Pestiziden, 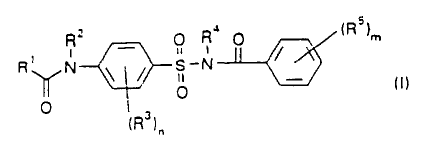 wobei in Formel (I)
R¹ Wasserstoff, einen Kohlenwasserstoffrest, einen Kohlenwasserstoffoxyrest oder einen Kohlenwasserstoffthiorest, wobei jeder der letztgenannten 3 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a}-R^{a} substituiert ist,
R² Wasserstoff oder (C₁-C₄)-Alkyl oder
R¹ und R²; zusammen mit der Gruppe der Formel -CO-N- den Rest eines 3- bis 8-gliedrigen gesättigten oder ungesättigten Rings und
R³, im Falle daß n =1 ist, oder die R³ unabhängig voneinander, im Falle daß n größer als 1 ist, jeweils Halogen, Cyano, Nitro, Amino, Hydroxy, Carboxy, Formyl, CONH₂, SO₂NH₂ oder einen Rest der Formel -Z^{b}-R^{b},
R⁴ Wasserstoff oder (C₁-C₄)-Alkyl,
R⁵, im Falle daß m = 1 ist, oder die R⁵ unabhängig voneinander, im Falle daß m größer als 1 ist, jeweils Halogen, Cyano, Nitro, Amino, Hydroxy, Carboxy, CHO, CONH₂, SO₂NH₂ oder einen Rest der Formel -Z^{c}-R^{c},
R^{a} einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)-alkyl]-amino substituiert ist, oder einen Alkylrest, in dem mehrere nicht benachbarte CH₂-Gruppen jeweils durch ein Sauerstoffatom ersetzt sind,
R^{b},R^{c} unabhängig voneinander einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Phosphoryl, Halogen-(C₁-C₄)-alkoxy, Mono- und Di-[(C₁-C₄)-alkyl]-amino substituiert ist, oder einen Alkylrest, in dem mehrere nicht benachbarte CH₂-Gruppen jeweils durch ein Sauerstoffatom ersetzt sind,
Z^{a} eine divalente Gruppe der Formel -O-, -S-, -CO-, -CS-, -CO-O-, -CO-S-, -O-CO-, -S-CO-, -SO-, -SO₂-, -NR*-, -CO-NR*-, -NR*-CO-, -SO₂-NR*- oder -NR*-SO₂-, wobei die rechts angegebene Bindung der jeweiligen divalenten Gruppe die Bindung zum Rest R^{a} ist und wobei die R* in den letztgenannten 5 Resten unabhängig voneinander jeweils H, (C₁-C₄)-Alkyl oder Halo-(C₁-C₄)-alkyl bedeuten,
Z^{b},Z^{c} unabhängig voneinander eine direkte Bindung oder eine divalente Gruppe der Formel -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*- oder -NR*-CO-, wobei die rechts angegebene Bindung der jeweiligen divalenten Gruppe die Bindung zum Rest R^{b} bzw. R^{c} ist und wobei die R* in den letztgenannten 5 Resten unabhängig voneinander jeweils H, (C₁-C₄)-Alkyl oder Halo-(C₁-C₄)-alkyl bedeuten,
n eine ganze Zahl von 0 bis 4 und
m eine ganze Zahl von 0 bis 5
bedeuten.

2. Verwendung nach Anspruch 1, **dadurch gekennzeichnet, daß** in Formel (I)
R¹ Wasserstoff, (C₁-C₁₂)-Alkyl, (C₂-C₈)-Alkenyl, (C₂-C₈)-Alkinyl, (C₃-C₈)-Cycloalkyl, (C₃-C₈)-Cycloalkenyl, (C₁-C₈)-Alkoxy, (C₂-C₈)-Alkenyloxy, (C₂-C₈)-Alkinyloxy, (C₃-C₈)-Cycloalkoxy, (C₃-C₈)-Cycloalkenyloxy, (C₁-C₈)-Alkylthio, (C₂-C₈)-Alkenylthio, (C₂-C₈)-Alkinylthio, (C₃-C₈)-Cycloalkylthio, (C₃-C₈)-Cycloalkenylthio oder Aryl, wobei jeder der letztgenannten 16 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a}-R^{a} substituiert ist,
R² Wasserstoff oder (C₁-C₄)-Alkyl oder
R¹ und R² zusammen mit der Gruppe der Formel -CO-N- den Rest eines 3- bis 8-gliedrigen gesättigten oder ungesättigten heterocyclischen Rings, der neben dem N-Atom der Gruppe der Formel -CO-N- noch 1 oder 2 Heteroatome aus der Gruppe N, O und S enthalten kann, und
R³, im Falle daß n = 1 ist, oder die R³ unabhängig voneinander, im Falle daß n größer als 1 ist, jeweils Halogen, Cyano, Nitro, Amino, Hydroxy, Phosphoryl, Carboxy, CHO, CONH₂, SO₂NH₂ oder einen Rest der Formel -Z^{b}-R^{b},
R⁴ Wasserstoff oder (C₁-C₄)-Alkyl,
R⁵, im Falle daß m = 1 ist, oder die R⁵ unabhängig voneinander, im Falle daß m größer als 1 ist, jeweils Halogen, Cyano, Nitro, Amino, Hydroxy, Carboxy, Phosphoryl, CHO, CONH₂ SO₂NH₂ oder einen Rest der Formel -Z^{c}-R^{c},
R^{a} (C₁-C₈)-Alkyl, (C₃-C₆)-Cycloalkyl, (C₂-C₈)-Alkenyl, (C₃-C₆)-Cycloalkenyl, (C₂-C₈)-Alkinyl, Phenyl oder einen Heterocyclylrest mit 3 bis 6 Ringatomen und 1 bis 3 Heteroringatomen aus der Gruppe N, O und S, wobei jeder der 7 letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)-alkyl]-amino substituiert ist, oder einen Alkylrest, in dem mehrere nicht benachbarte CH₂-Gruppen jeweils durch ein Sauerstoffatom ersetzt sind,
R^{b},R^{c} unabhängig voneinander (C₁-C₈)-Alkyl, (C₃-C₆)-Cycloalkyl, (C₂-C₈)-Alkenyl, (C₃-C₆)-Cycloalkenyl, (C₂-C₈)-Alkinyl, Phenyl oder einen Heterocyclylrest mit 3 bis 6 Ringatomen und 1 bis 3 Heteroringatomen aus der Gruppe N, O und S, wobei jeder der 7 letztgenannten Reste unsubstituiert oder durch einen oder mehrere gleiche oder verschiedene Reste aus der Gruppe Halogen, Cyano, Nitro, Amino, Hydroxy, Phosphoryl, Halogen-(C₁-C₄)-alkoxy, Mono- und Di-[(C₁-C₄)-alkyl]-amino substituiert ist, oder einen Alkylrest, in dem mehrere nicht benachbarte CH₂-Gruppen jeweils durch ein Sauerstoffatom ersetzt sind,
Z^{a} eine divalente Gruppe der Formel -O-, -S-, -CO-, -CS-, -CO-O-, -CO-S-, -O-CO-, -S-CO-, -SO-, -SO₂-, -NR*-, -CO-NR*- oder -NR*-CO-, wobei die rechts angegebene Bindung der jeweiligen divalenten Gruppe die Bindung zum Rest R^{a} ist und wobei die R* in den letztgenannten beiden Resten unabhängig voneinander jeweils H, (C₁-C₄)-Alkyl oder (C₁-C₄)-Haloalkyl bedeuten,
Z^{b},Z^{c} unabhängig voneinander eine direkte Bindung oder eine divalente Gruppe der Formel -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*- oder -NR*-CO-, wobei die rechts angegebene Bindung der jeweiligen divalenten Gruppe die Bindung zum Rest R^{b} bzw. R^{c} ist und wobei die R* in den letztgenannten 5 Resten unabhängig voneinander jeweils H, (C₁-C₄)-Alkyl oder Halo-(C₁-C₄)-Alkyl bedeuten,
bedeuten.

3. Verwendung nach Anspruch 1 oder 2, **dadurch gekennzeichnet, daß** in Formel (I)
R¹ Wasserstoff, (C₁-C₁₂)-Alkyl, (C₂-C₈)-Alkenyl, (C₂-C₈)-Alkinyl, (C₃-C₈)-Cycloalkyl, (C₃-C₈)-Cycloalkenyl, (C₁-C₈)-Alkoxy, (C₂-C₈)-Alkenyloxy, (C₂-C8)-Alkinyloxy, (C₃-C₈)-Cycloalkoxy, (C₃-C₈)-Cycloalkenyloxy, (C₁-C₆)-Alkylthio, (C₂-C₈)-Alkenylthio, (C₂-C₈)-Alkinylthio, (C₃-C₈)-Cycloalkylthio, (C₃-C₈)-Cycloalkenylthio oder Phenyl, wobei jeder der vorstehenden C-haltigen Reste unsubstituiert oder durch einen oder mehrere gleiche oder verschiedene Substituenten aus der Gruppe Halogen, Nitro, Cyano, Amino, Hydroxy, (C₁-C₈)-Alkoxy, worin eine oder mehrere nicht benachbarte CH₂-Gruppen durch Sauerstoff ersetzt sein können, (C₁-C₈)-Alkylthio, (C₁-C₆)-Alkylsulfinyl, (C₁-C₆)-Alkylsulfonyl, (C₂-C₈)-Alkenyloxy, (C₂-C₈)-Alkenylthio, (C₂-C₈)-Alkinyloxy, (C₂-C₈)-Alkinylthio, (C₃-C₇)-Cycloalkyl, (C₃-C₇)-Cycloalkenyl, (C₃-C₇)-Cycloalkoxy, (C₃-C₇)-Cycloalkenyloxy, Mono- und Di-[(C₁-C₄)-alkyl]-amino, [(C₁-C₈)-Alkoxy]-carbonyl, [(C₂-C₈)-Alkenyloxy]-carbonyl, [(C₂-C₈)-Alkinyloxy]-carbonyl, [(C₁-C₈)-Alkylthio]-carbonyl, [(C₁-C₈)-Alkyl]-carbonyl, [(C₂-C₈)-Alkenyl]-carbonyl, [(C₂-C₈)-Alkinyl]-carbonyl, Phenyl, Phenyl-(C₁-C₆)-alkoxy, Heterocyclyl mit 3 bis 6 Ringatomen und 1 bis 3 Heteroringatomen aus der Gruppe N, O und S und im Falle cyclischer Reste auch (C₁-C₆)-Alkyl substituiert sind, wobei jeder der 25 letztgenannten Reste unsubstituiert oder durch einen oder mehrere Reste aus der Gruppe Halogen, Nitro, Amino, Cyano und Hydroxy substituiert ist,
R² Wasserstoff oder (C₁-C₄)-Alkyl oder
R¹ und R² zusammen mit der Gruppe der Formel -CO-N- den Rest eines 5- bis 6-gliedrigen gesättigten oder ungesättigten heterocyclischen Rings, der neben dem N-Atom der Gruppe der Formel -CO-N- noch 1 Heteroatom aus der Gruppe N, O und S enthalten kann, und
R³, R⁵ jeweils gleiche oder verschiedene Reste, welche unabhängig voneinander Halogen, Nitro, Amino, Hydroxy, Cyano, Sulfamoyl, (C₁-C₈)-Alkyl, (C₃-C₆)-Cycloalkyl, (C₂-C₈)-Alkenyl, (C₂-C₈)-Alkinyl, (C₁-C₈)-Alkoxy, (C₂-C₈)-Alkenyloxy, (C₂-C₈)-Alkinyloxy, Mono- oder Di-[(C₁-C₄)-Alkyl]-aminosulfonyl, (C₁-C₈)-Alkylthio, (C₁-C₈)-Alkylsulfinyl, (C₁-C₈)-Alkylsulfonyl, (C₁-C₈)-Alkoxycarbonyl, (C₁-C₈)-Alkylthiocarbonyl, (C₁-C₈)-Alkylcarbonyl, wobei jeder der letztgenannten 15 Reste unsubstituiert oder durch einen oder mehrere gleiche oder verschiedene Reste aus der Gruppe Halogen, Halogen-(C₁-C₆)-alkoxy, Phosphoryl, Nitro, Amino, Cyano, Hydroxy, (C₁-C₈)-Alkoxy, worin eine oder mehrere nicht benachbarte CH₂-Gruppen durch Sauerstoff ersetzt sein können, und im Falle cyclischer Reste auch (C₁-C₄)-Alkyl und (C₁-C₄)-Haloalkyl substituiert ist,
bedeuten.

4. Verwendung nach einem der Ansprüche 1 bis 3, **dadurch gekennzeichnet daß** in Formel (I)
R¹ Wasserstoff, (C₁-C₈)-Alkyl, (C₂-C₆)-Alkenyl, (C₂-C₆)-Alkinyl, (C₃-C₆)-Cycloalkyl, (C₅-C₆)-Cycloalkenyl, (C₁-C₆)-Alkoxy, (C₂-C₆)-Alkenyloxy, (C₂-C₆)-Alkinyloxy, (C₃-C₆)-Cycloalkoxy, (C₅-C₆)-Cycloalkenyloxy, (C₁-C₆)-Alkylthio, (C₂-C₆)-Alkenylthio, (C₂-C₆)-Alkinylthio, (C₃-C₆)-Cycloalkylthio, (C₅-C₆)-Cycloalkenylthio oder Phenyl, wobei jeder der 16 letztgenannten Reste unsubstituiert oder durch einen oder mehrere gleiche oder verschiedene Substituenten aus der Gruppe Halogen, Cyano, (C₁-C₆)-Alkoxy, (C₁-C₆)-Alkylthio, (C₁-C₄)-Alkylsulfinyl, (C₁-C₄)-Alkylsulfonyl, (C₂-C₄)-Alkenyloxy, (C₂-C₄)-Alkenylthio, (C₂-C₄)-Alkinyloxy, (C₂-C₄)-Alkinylthio, (C₃-C₆)-Cycloalkyl, (C₅-C₆)-Cycloalkenyl, (C₃-C₆)-Cycloalkoxyl, (C₅-C₆)-Cycloalkenyloxy, Mono- und Di-[(C₁-C₄)-alkyl]-amino, [(C₁-C₆)-Alkoxy]-carbonyl, [(C₁-C₆)-Alkylthio]-carbonyl, [(C₁-C₆)-Alkyl]-carbonyl, Phenyl, Phenyl-(C₁-C₄)-alkoxy, Heterocyclyl mit 5 bis 6 Ringatomen und 1 bis 3 Heteroringatomen aus der Gruppe N, O und S und im Falle cyclischer Reste auch (C₁-C₄)-Alkyl substituiert ist, wobei jeder der 21 letztgenannten Reste unsubstituiert oder durch einen oder mehrere Reste aus der Gruppe Halogen und Cyano und im Falle cyclischer Reste auch (C₁-C₄)-Alkyl substituiert ist,
R² Wasserstoff oder (C₁-C₄)-Alkyl oder
R¹ und R² zusammen mit der Gruppe der Formel -CO-N- den Rest eines 5- bis 6-gliedrigen gesättigten oder ungesättigten heterocyclischen Rings, der neben dem N-Atom der Gruppe der Formel -CO-N- kein weiteres Heteroringatom enthält, und
R³, R⁵ jeweils gleiche oder verschiedene Reste, welche unabhängig voneinander Halogen, Nitro, Amino, Hydroxy, Cyano, Sulfamoyl, (C₁-C₆)-Alkyl, (C₃-C₆)-Cycloalkyl, (C₂-C₆)-Alkenyl, (C₂-C₆)-Alkinyl, (C₁-C₆)-Alkoxy, (C₂-C₈)-Alkenyloxy, (C₂-C₈)-Alkinyloxy, Mono- und Di-[(C₁-C₄)-Alkyl]-aminosulfonyl, (C₁-C₆)-Alkylthio, (C₁-C₈)-Alkylsulfinyl, (C₁-C₆)-Alkylsulfonyl, (C₁-C₆)-Alkoxycarbonyl, (C₁-C₆)-Alkylthiocarbonyl, (C₁-C₆)-Alkylcarbonyl, wobei jeder der letztgenannten 15 Reste unsubstituiert oder durch einen oder mehrere gleiche oder verschiedene Reste aus der Gruppe Halogen, Halogen-(C₁-C₄)-alkoxy, Cyano, (C₁-C₆)-Alkoxy und im Falle cyclischer Reste auch (C₁-C₄)-Alkyl und (C₁-C₄)-Haloalkyl substituiert ist,
bedeuten.

5. Verwendung nach einem der Ansprüche 1 bis 4, **dadurch gekennzeichnet, daß** in Formel (I)
R¹ Wasserstoff, (C₁-C₆)-Alkyl, (C₃-C₆)-Cycloalkyl, (C₂-C₆)-Alkenyl, (C₅-C₆)-Cycloalkenyl, (C₁-C₆)-Alkoxy oder Phenyl, wobei jeder der 6 letztgenannten Reste unsubstituiert oder durch einen oder mehrere gleiche oder verschiedene Reste aus der Gruppe Halogen, (C₁-C₈)-Alkoxy, worin eine oder mehrere CH₂-Gruppen durch Sauerstoff ersetzt sein können, (C₁-C₆)-Halogenalkoxy, (C₁-C₂)-Alkylsulfinyl, (C₁-C₂)-Alkylsulfonyl, (C₃-C₆)-Cycloalkyl, (C₁-C₄)-Alkoxycarbonyl, (C₁-C₄)-Alkylcarbonyl und Phenyl und im Falle im Falle cyclischer Reste auch (C₁-C₄)-Alkyl und (C₁-C₄)-Haloalkyl substituiert ist,
R² Wasserstoff,
R³ Halogen, Halogen-(C₁-C₄)-alkyl, Halogen-(C₁-C₄)-alkoxy, (C₁-C₄)-Alkyl, (C₁-C4)-Alkoxy, (C₁-C₄)-Alkylsulfonyl, (C₁-C₄)-Alkoxycarbonyl oder (C₁-C₄)-Alkylcarbonyl,
R⁴ Wasserstoff,
R⁵ Halogen, (C₁-C₄)-Alkyl, Halogen-(C₁-C₄)-alkyl, Halogen-(C₁-C₄)-alkoxy, (C₃-C₆)-Cycloalkyl, Phenyl, (C₁-C₄)-Alkoxy, Cyano, (C₁-C₄)-Alkylthio, (C₁-C₄)-Alkylsulfinyl, (C₁-C₄)-Alkylsulfonyl, (C₁-C₄)-Alkoxycarbonyl oder (C₁-C₄)-Alkylcarbonyl,
n 0, 1 oder 2 und
m 1 oder 2 bedeuten.

6. Verwendung nach einem der Ansprüche 1 bis 5, **dadurch gekennzeichnet, daß** in Formel (I)
R¹ Wasserstoff, (C₁-C₆)-Alkyl oder (C₃-C₆)-Cycloalkyl, wobei jeder der letztgenannten 2 Reste unsubstituiert oder durch einen oder mehrere Substituenten aus der Gruppe Halogen, (C₁-C₄)-Alkoxy, Halogen-(C₁-C₆)-alkoxy und (C₁-C₄)-Alkylthio und im Falle cyclischer Reste auch (C₁-C₄)-Alkyl und (C₁-C₄)-Haloalkyl substituiert ist,
R² Wasserstoff,
R³ Halogen, Halogen-(C₁-C₄)-alkyl, Halogen-(C₁-C₄)-alkoxy, (C₁-C₄)-Alkyl, (C₁-C₄)-Alkoxy, (C₁-C₄)-Alkylsulfonyl, (C₁-C₄)-Alkoxycarbonyl oder (C₁-C₄)-Alkylcarbonyl,
R⁴ Wasserstoff,
R⁵ Halogen, (C₁-C₄)-Alkyl, Halogen-(C₁-C₄)-alkyl, Halogen-(C₁-C₄)-alkoxy, (C₃-C₆)-Cycloalkyl, Phenyl, (C₁-C₄)-Alkoxy, Cyano, (C₁-C₄)-Alkylthio, (C₁-C₄)-Alkylsulfinyl, (C₁-C₄)-Alkylsulfonyl, (C₁-C₄)-Alkoxycarbonyl oder (C₁-C₄)-Alkylcarbonyl,
n 0, 1 oder 2 und
m 1 oder 2 bedeuten.

7. Verbindungen der Formel (I) und deren Salze, wie sie in einem der Ansprüche 1 bis 6 definiert sind, ausgenommen Verbindungen der Formel (I), worin
a) R² =H, R³ =H, n = 0 und
a1)
R¹ = CH₃ und
m = 0 oder (R⁵)ₘ =2-, 3- oder 4-CH₃, 4-C₂H₅, 4-n-C₃H₇, 4-i-C₃H₇, 4-OCH₃, 4-i-OC₃H₇, 4-NH₂, 4-Cl, 4-NO₂, 2-COOH, 2,3-(CH₃)₂, 2,4-(CH₃)₂, 3,4-(CH₃)₂, 2,5-(CH₃)₂, 2,4,5-(CH₃)₃, 2,4,6-(CH₃)₃, 2,3,4,5,6-(CH₃)₅, 3-CH₃-4-OCH₃, 3-CH₃-4-SCH₃, 2,4-(OCH₃)₂, 2,5-(OCH₃)₂, 3,4,5-(OCH₃)₃, 2-OCH₃-4-NH₂, 2-OCH₃-4-NO₂, 2-OH-4-NO₂ oder 2-OH-4-NH₂,
a2) R¹ = H, CH₃, n-C₃H₇, n-C₄H₉, n-C₅H₁₁, n-C₆H₁₃, Cyclohexyl, 2-Methylphenyl oder n-C₆H₅-CH=CH- und jeweils (R⁵)ₘ = 2-CH₃,
a3)
R¹ = n-C₅H₁₁ und
m = 0 oder (R⁵)ₘ = 2-CH₃, 3-NO₂ oder 4-NO₂,
a4) R¹ = n-C₉H₁₉ und m = 0,
a5) R¹ = OCH₃, (R⁵)ₘ =2-i-OC₃H₇ oder 4-i-OC₃H₇,
a6) R¹ = OC₂H₅, (R⁵)ₘ= 2-OH, 2-OCH₃, 2-COOH, 3,5-(CH₃)₂,
a7)
R¹ = CH₂CH₂COOH und
m = 0 oder (R⁵)ₘ = 4-i-OC₃H₇,
a8) R¹ = CH = CHCOOH und (R⁵)ₘ = 2-CH₃ oder 4-i-OC₃H₇,
a9) R¹ = 4-Methoxyphenyl und (R⁵)ₘ = 4-OCH₃,
a10) R¹ = 4-Nitrophenyl und (R⁵)ₘ = 4-NO₂,
a11) R¹ = CH₂Cl, R²=R⁴=H und m=n=0,
a12)
R¹ = 3,5-Dimethyl-1-phenyl-pyrazol-4-yl oder 2,3-Dimethyl-1-phenyl-5-oxo-pyrazol-4-yl und
(R⁵)ₘ= 4-i-OC₃H₇,
a13)
R¹ = C₁₁H₂₃, CH₂Cl, CH₂Br, CH₂l, CHCl₂, CCl₃ oder CH₂F und
(R⁵)ₘ = 3,4-(CH₃)₂; siehe Chem. Abstr. 55: 461g; oder
b) R¹ =H, R² =H, R⁴ =CH₃, n = m=0,
c) R¹ = -C(CH₃)=CH₂, R²=R⁴=H, n=0 und (R⁵)ₘ = 4-CH₃,
d) R¹ = Phenyl, R² = R⁴ = H, m=0 und n=0 oder (R³)ₙ= 3-Phenylcarbonyloxy,
e) N-([(4-Benzoylsulfamoyl)phenyl]-phthalimid,
f) R¹ = 2-(2-Carboxyphenyl)phenyl, R²=R⁴=H, n=0 und m=0 oder (R⁵)ₘ= 4-NO₂,
g) R¹ = 2-Carboxyphenyl, R²=R⁴=H, n=0 und m=0 oder
h) R¹ = CH₃, R²=H, R⁴=CH₃ und n=m=0
bedeuten.

8. Verfahren zur Herstellung von Verbindungen der Formel (I) oder,deren Salzen, wie sie in Anspruch 7 definiert sind, **dadurch gekennzeichnet, daß** man
1. eine Verbindung der Formel (II), 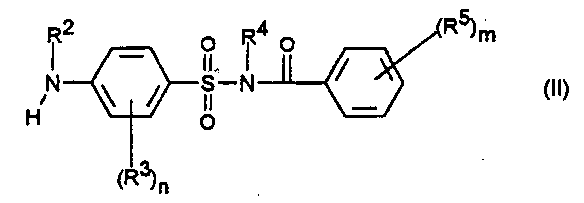 worin R², R³, R⁴, R⁵, n und m wie in Formel (I) definiert sind, mit einem Acylierungsmittel der Formel R¹-CO-Nuc, worin Nuc eine Abgangsgruppe ist und R¹ wie in Formel (I) definiert ist, umsetzt oder
2. eine Verbindung der Formel (III), 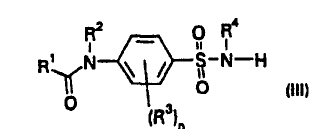 worin R¹, R², R³, R⁴ und n wie in Formel (I) definiert sind, mit einem Benzoylhalogenid der Formel (IV) umsetzt, 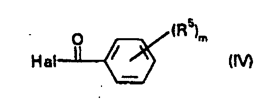 worin R⁵ und m die in Formel (I) gegebenen Bedeutungen haben.

9. Pestizide Mittel mit einem wirksamen Gehalt an
A) einem oder mehreren pestiziden Wirkstoffen, welche bei Anwendung ohne Safener phytotoxische Schäden an Kulturpflanzen ergeben können,
B) einem oder mehreren Safenern der Formel (I) oder dessen Salz, wie sie gemäß einem der Ansprüche 1 bis 7 definiert sind.

10. Verfahren zum Schutz von Kulturpflanzen vor phytotoxischen Nebenwirkungen von Pestiziden, das **dadurch gekennzeichnet ist, daß** eine wirksame Menge mindestens einer Verbindung der Formel (I) oder dessen Salz, welche gemäß einem der Ansprüche 1 bis 7 definiert sind, als Safener vor, nach oder gleichzeitig mit einem pestiziden Wirkstoff auf die Pflanzen, Pflanzensamen oder die Anbaufläche appliziert wird.

## Claims

1. The use of compounds of the formula (I) or salts thereof as safeners for protecting crop plants against phytotoxic side effects of pesticides, 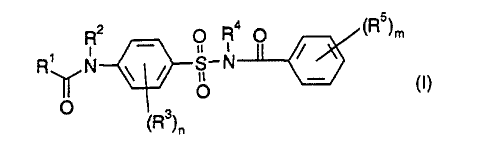 where, in formula (I),
R¹ is hydrogen, a hydrocarbon radical, a hydrocarbonoxy radical or a hydrocarbonthio radical, each of the last-mentioned 3 radicals being unsubstituted or substituted by one or more identical or different radicals selected from the group consisting of halogen, cyano, nitro, amino, hydroxyl, carboxyl, formyl, carboxamide, sulfonamide and radicals of the formula -Z^{a}-R^{a},
R² is hydrogen or (C₁-C₄)-alkyl, or
R¹ and R² together with the group of the formula -CO-N- are the radical of a 3- to 8-membered saturated or unsaturated ring and
R³, in the event that n=1, or the R³ radicals independently of one another, in the event that n is greater than 1, is, or are, in each case halogen, cyano, nitro, amino, hydroxyl, carboxyl, formyl, CONH₂, SO₂NH₂ or a radical of the formula -Z^{b}-R^{b},
R⁴ is hydrogen or (C₁-C₄)-alkyl,
R⁵, in the event that m=1, or the R⁵ radicals independently of one another, in the event that m is greater than 1, is, or are, in each case halogen, cyano, nitro, amino, hydroxyl, carboxyl, CHO, CONH₂, SO₂NH₂ or a radical of the formula -Z^{c}-R^{c},
R^{a} is a hydrocarbon radical or a heterocyclyl radical, each of the two last-mentioned radicals being unsubstituted or substituted by one or more identical or different radicals selected from the group consisting of halogen, cyano, nitro, amino, hydroxyl, mono- and di-[(C₁-C₄)-alkyl]amino, or an alkyl radical in which more than one non-adjacent CH₂ group is in each case replaced by an oxygen atom.
R^{b},R^{c} independently of one another are a hydrocarbon radical or a heterocyclyl radical, each of the two last-mentioned radicals being unsubstituted or substituted by one or more identical or different radicals selected from the group consisting of halogen, cyano, nitro, amino, hydroxyl, phosphoryl, halo-(C₁-C₄)-alkoxy, mono- and di-[(C₁-C₄)-alkyl]amino, or an alkyl radical in which more than one non-adjacent CH₂ group is in each case replaced by an oxygen atom,
Z^{a} is a divalent group of the formula -O-, -S-, -CO-, -CS-, -CO-O-, -CO-S-, -O-CO-, -S-CO-, -SO-, -SO₂-, -NR*-, -CO-NR*-, -NR*-CO-, -SO₂-NR*- or -NR*-SO₂-, the bond shown on the right of the divalent group in question being the bond to the radical R^{a} and the R* radicals in the last-mentioned 5 radicals independently of one another being in each case H, (C₁-C₄)-alkyl or halo-(C₁-C₄)-alkyl,
Z^{b},Z^{c} independently of one another are a direct bond or divalent group of the formula -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*- or -NR*-CO-, the bonds given on the right of the divalent group in question being the bond to the radical R^{b} or R^{c}, and the R* radicals in the last-mentioned 5 radicals independently of one another in each case being H, (C₁-C₄)-alkyl or halo-(C₁-C₄)-alkyl,
n is an integer from 0 to 4, and
m is an integer from 0 to 5.

2. The use as claimed in claim 1, wherein, in formula (I),
R¹ is hydrogen, (C₁-C₁₂)-alkyl, (C₂-C₈)-alkenyl, (C₂-C₈)-alkynyl, (C₃-C₈)-cycloalkyl, (C₃-C₈)-cycloalkenyl, (C₁-C₈)-alkoxy, (C₂-C₈)-alkenyloxy, (C₂-C₈)-alkynyloxy, (C₃-C₈)-cycloalkoxy, (C₃-C₈)-cycloalkenyloxy, (C₁-C₈)-alkylthio, (C₂-C₈)-alkenylthio, (C₂-C₈)-alkynylthio, (C₃-C₈)-cycloalkylthio, (C₃-C₈)-cycloalkenylthio or aryl, each of the last-mentioned 16 radicals being unsubstituted or substituted by one or more identical or different radicals selected from the group consisting of halogen, cyano, nitro, amino, hydroxyl, carboxyl, formyl, carboxamide, sulfonamide and radicals of the formula -Z^{a}-R^{a,}
R² is hydrogen or (C₁-C₄)-alkyl or
R¹ and R² together with the group of the formula -CO-N- are the radical of a 3- to 8-membered saturated or unsaturated heterocyclic ring which, in addition to the nitrogen atom of the group of the formula -CO-N-, can additionally contain 1 or 2 hetero atoms selected from the group consisting of N, O and S, and
R³, in the event that n=1, or the R³ radicals independently of one another, in the event that n is greater than 1, is, or are, in each case halogen, cyano, nitro, amino, hydroxyl, phosphoryl, carboxyl, CHO, CONH₂, SO₂NH₂ or a radical of the formula -Z^{b}-R^{b},
R⁴ is hydrogen or (C₁-C₄)-alkyl,
R⁵, in the event that m=1, or the R⁵ radicals independently of one another, in the event that m is greater than 1, is, or are, in each case halogen, cyano, nitro, amino, hydroxyl, carboxyl, phosphoryl, CHO, CONH₂, SO₂NH₂ or a radical of the formula -Z^{c}-R^{c},
R^{a} is (C₁-C₈)-alkyl, (C₃-C₆)-cycloalkyl, (C₂-C₈)-alkenyl, (C₃-C₆)-cycloalkenyl, (C₂-C₈)-alkynyl, phenyl or a heterocyclyl radical having 3 to 6 ring atoms and 1 to 3 hetero ring atoms selected from the group consisting of N, O and S, each of the 7 last-mentioned radicals being unsubstituted or substituted by one or more identical or different radicals selected from the group consisting of halogen, cyano, nitro, amino, hydroxyl, mono- and di-[(C₁-C₄)-alkyl]amino, or an alkyl radical in which more than one non-adjacent CH₂ group is in each case replaced by an oxygen atom,
R^{b},R^{c} independently of one another are (C₁-C₈)-alkyl, (C₃-C₆)-cycloalkyl, (C₂-C₈)-alkenyl, (C₃-C₆)-cycloalkenyl, (C₂-C₈)-alkynyl, phenyl or a heterocyclyl radical having 3 to 6 ring atoms and 1 to 3 hetero ring atoms selected from the group consisting of N, O and S, each of the 7 last-mentioned radicals being unsubstituted or substituted by one or more identical or different radicals selected from the group consisting of halogen, cyano, nitro, amino, hydroxyl, phosphoryl, halo-(C₁-C₄)-alkoxy, mono- and di-[(C₁-C₄)-alkyl]amino, or an alkyl radical in which more than one non-adjacent CH₂ group is in each case replaced by an oxygen atom,
Z^{a} is a divalent group of the formula -O-, -S-, -CO-, -CS-, -CO-O-, -CO-S-, -O-CO-, -S-CO-, -SO-, -SO₂-, -NR*-, -CO-NR*- or -NR*-CO-, the bond shown on the right of the divalent group in question being the bond to the radical R^{a}, and the R* radicals in the last-mentioned two radicals independently of one another being in each case H, (C₁-C₄)-alkyl or (C₁-C₄)-haloalkyl,
Z^{b},Z^{c} independently of one another being a direct bond or a divalent group of the formula -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*- or -NR*-CO -, the bond shown on the right of the divalent group in question being the bond to the radical R^{b} or R^{c}, respectively, and the R* radicals in the last-mentioned 5 radicals independently of one another being in each case H, (C₁-C₄)-alkyl or halo-(C₁-C₄)-alkyl.

3. The use as claimed in claim 1 or 2, wherein, in formula (I),
R¹ is hydrogen, (C₁-C₁₂)-alkyl, (C₂-C₈)-alkenyl, (C₂-C₈)-alkynyl, (C₃-C₈)-cycloalkyl, (C₃-C₈)-cycloalkenyl, (C₁-C₈)-alkoxy, (C₂-C₈)-alkenyloxy, (C₂-C₈)-alkynyloxy, (C₃-C₈)-cycloalkoxy, (C₃-C₈)-cycloalkenyloxy, (C₁-C₆)-alkylthio, (C₂-C₈)-alkenylthio, (C₂-C₈)-alkynylthio, (C₃-C₈)-cycloalkylthio, (C₃-C₈)-cycloalkenylthio or phenyl, where each of the above carbon-containing radicals is unsubstituted or substituted by one or more identical or different substituents selected from the group consisting of halogen, nitro, cyano, amino, hydroxyl, (C₁-C₈)-alkoxy - where one or more non-adjacent CH₂ groups can be replaced by oxygen -, (C₁-C₈)-alkylthio, (C₁-C₆)-alkylsulfinyl, (C₁-C₆)-alkylsulfonyl, (C₂-C₈)-alkenyloxy, (C₂-C₈)-alkenylthio, (C₂-C₈)-alkynyloxy, (C₂-C₈)-alkynylthio, (C₃-C₇)-cycloalkyl, (C₃-C₇)-cycloalkenyl, (C₃-C₇)-cycloalkoxy, (C₃-C₇)-cycloalkenyloxy, mono- and di-[(C₁-C₄)-alkyl]amino, [(C₁-C₈)-alkoxy]carbonyl, [(C₂-C₈)-alkenyloxy]carbonyl, [(C₂-C₈)-alkynyloxy]carbonyl, [(C₁-C₈)-alkylthio]carbonyl, [(C₁-C₈)-alkyl]carbonyl, [(C₂-C₈)-alkenyl]carbonyl, [(C₂-C₈)-alkynyl]carbonyl, phenyl, phenyl-(C₁-C₆)-alkoxy and heterocyclyl having 3 to 6 ring atoms and 1 to 3 hetero ring atoms selected from the group consiting of N, O and S and, in the case of cyclic radicals, also (C₁-C₆)-alkyl, each of the 25 last-mentioned radicals being unsubstituted or substituted by one or more radicals selected from the group consisting of halogen, nitro, amino, cyano and hydroxyl,
R² is hydrogen or (C₁-C₄)-alkyl or
R¹ and R² together with the group of the formula -CO-N- are the radical of a 5- to 6-membered saturated or unsaturated heterocyclic ring which, in addition to the nitrogen atom of the group of the formula -CO-N-, can also contain 1 hetero atom selected from the group consisting of N, O and S, and
R³, R⁵ in each case are identical or different radicals which, independently of one another, are halogen, nitro, amino, hydroxyl, cyano, sulfamoyl, (C₁-C₈)-alkyl, (C₃-C₆)-cycloalkyl, (C₂-C₈)-alkenyl, (C₂-C₈)-alkynyl, (C₁-C₈)-alkoxy, (C₂-C₈)-alkenyloxy, (C₂-C₈)-alkynyloxy, mono- or di-[(C₁-C₄)-alkyl]aminosulfonyl, (C₁-C₈)-alkylthio, (C₁-C₈)-alkylsulfinyl, (C₁-C₈)-alkylsulfonyl, (C₁-C₈)-alkoxycarbonyl, (C₁-C₈)-alkylthiocarbonyl, (C₁-C₈)-alkylcarbonyl, it being possible for each of the last-mentioned 15 radicals to be unsubstituted or substituted by one or more identical or different radicals selected from the group consisting of halogen, halo-(C₁-C₆)-alkoxy, phosphoryl, nitro, amino, cyano, hydroxyl, (C₁-C₈)-alkoxy, in which one or more non-adjacent CH₂ groups can be replaced by oxygen and, in the case of cyclic radicals, also (C₁-C₄)-alkyl and (C₁-C₄)-haloalkyl.

4. The use as claimed in any of claims 1 to 3, wherein, in formula (I),
R¹ is hydrogen, (C₁-C₈)-alkyl, (C₂-C₆)-alkenyl, (C₂-C₆)-alkynyl, (C₃-C₆)-cycloalkyl, (C₅-C₆)-cycloalkenyl, (C₁-C₆)-alkoxy, (C₂-C₆)-alkenyloxy, (C₂-C₆)-alkynyloxy, (C₃-C₆)-cycloalkoxy, (C₅-C₆)-cycloalkenyloxy, (C₁-C₆)-alkylthio, (C₂-C₆)-alkenylthio, (C₂-C₆)-alkynylthio, (C₃-C₆)-cycloalkylthio, (C₅-C₆)-cycloalkenylthio or phenyl, each of the 16 last-mentioned radicals being unsubstituted or substituted by one or more identical or different substituents selected from the group consisting of halogen, cyano, (C₁-C₆)-alkoxy, (C₁-C₆)-alkylthio, (C₁-C₄)-alkylsulfinyl, (C₁-C₄)-alkylsulfonyl, (C₂-C₄)-alkenyloxy, (C₂-C₄)-alkenylthio, (C₂-C₄)-alkynyloxy, (C₂-C₄)-alkynylthio, (C₃-C₆)-cycloalkyl, (C₅-C₆)-cycloalkenyl, (C₃-C₆)-cycloalkoxy, (C₅-C₆)-cycloalkenyloxy, mono- and di-[(C₁-C₄)-alkyl]amino, [(C₁-C₆)-alkoxy]-carbonyl, [(C₁-C₆)-alkylthio]carbonyl, [(C₁-C₆)-alkyl]carbonyl, phenyl, phenyl-(C₁-C₄)-alkoxy, heterocyclyl having 5 to 6 ring atoms and 1 to 3 hetero ring atoms selected from the group consisting of N, O and S and, in the case of cyclic radicals, also (C₁-C₄)-alkyl, each of the 21 last-mentioned radicals being unsubstituted or substituted by one or more radicals selected from the group consisting of halogen, cyano and, in the case of cyclic radicals, also (C₁-C₄)-alkyl,
R² is hydrogen or (C₁-C₄)-alkyl or
R¹ and R² together with the group of the formula -CO-N- are the radical of a 5- to 6-membered saturated or unsaturated heterocyclic ring which, in addition to the nitrogen atom of the group of the formula -CO-N-, contains no further hetero ring atom, and
R³, R⁵ in each case are identical or different radicals which, independently of one another, are halogen, nitro, amino, hydroxyl, cyano, sulfamoyl, (C₁-C₆)-alkyl, (C₃-C₆)-cycloalkyl, (C₂-C₆)-alkenyl, (C₂-C₆)-alkynyl, (C₁-C₆)-alkoxy, (C₂-C₆)-alkenyloxy, (C₂-C₆)-alkynyloxy, mono- and di-[(C₁-C₄)-alkyl]aminosulfonyl, (C₁-C₆)-alkylthio, (C₁-C₈)-alkylsulfinyl, (C₁-C₆)-alkylsulfonyl, (C₁-C₆)-alkoxycarbonyl, (C₁-C₆)-alkylthiocarbonyl, (C₁-C₆)-alkylcarbonyl, each of the last-mentioned 15 radicals being unsubstituted or substituted by one or more identical or different radicals selected from the group consisting of halogen, halo-(C₁-C₄)-alkoxy, cyano, (C₁-C₆)-alkoxy and, in the case of cyclic radicals, also (C₁-C₄)-alkyl and (C₁-C₄)-haloalkyl.

5. The use as claimed in any of claims 1 to 4, wherein, in formula (I),
R¹ is hydrogen, (C₁-C₆)-alkyl, (C₃-C₆)-cycloalkyl, (C₂-C₆)-alkenyl, (C₅-C₆)-cycloalkenyl, (C₁-C₆)-alkoxy or phenyl, each of the 6 last-mentioned radicals being unsubstituted or substituted by one or more identical or different radicals selected from the group consisting of halogen, (C₁-C₆)-alkoxy - where one or more CH₂ groups can be replaced by oxygen -, (C₁-C₆)-haloalkoxy, (C₁-C₂)-alkylsulfinyl, (C₁-C₂)-alkylsulfonyl, (C₃-C₆)-cycloalkyl, (C₁-C₄)-alkoxycarbonyl, (C₁-C₄)-alkylcarbonyl and phenyl and, in the case of cyclic radicals, also (C₁-C₄)-alkyl and (C₁-C₄)-haloalkyl,
R² is hydrogen,
R³ is halogen, halo-(C₁-C₄)-alkyl, halo-(C₁-C₄)-alkoxy, (C₁-C₄)-alkyl, (C₁-C₄)-alkoxy, (C₁-C₄)-alkylsulfonyl, (C₁-C₄)-alkoxycarbonyl or (C₁-C₄)-alkylcarbonyl,
R⁴ is hydrogen,
R⁵ is halogen, (C₁-C₄)-alkyl, halo-(C₁-C₄)-alkyl, halo-(C₁-C₄)-alkoxy, (C₃-C₆)-cycloalkyl, phenyl, (C₁-C₄)-alkoxy, cyano, (C₁-C₄)-alkylthio, (C₁-C₄)-alkylsulfinyl, (C₁-C₄)-alkylsulfonyl, (C₁-C₄)-alkoxycarbonyl or (C₁-C₄)-alkylcarbonyl,
n is 0, 1 or 2 and
m is 1 or 2.

6. The use as claimed in any of claims 1 to 5, wherein, in formula (I),
R¹ is hydrogen, (C₁-C₆)-alkyl or (C₃-C₆)-cycloalkyl, each of the last-mentioned 2 radicals being unsubstituted or substituted by one or more substituents selected from the group consisting of halogen, (C₁-C₄)-alkoxy, halo-(C₁-C₆)-alkoxy and (C₁-C₄)-alkylthio and, in the case of cyclic radicals, also (C₁-C₄)-alkyl and (C₁-C₄)-haloalkyl,
R² is hydrogen,
R³ is halogen, halo-(C₁-C₄)-alkyl, halo-(C₁-C₄)-alkoxy, (C₁-C₄)-alkyl, (C₁-C₄)-alkoxy, (C₁-C₄)-alkylsulfonyl, (C₁-C₄)-alkoxycarbonyl or (C₁-C₄)-alkylcarbonyl,
R⁴ is hydrogen,
R⁵ is halogen, (C₁-C₄)-alkyl, halo-(C₁-C₄)-alkyl, halo-(C₁-C₄)-alkoxy, (C₃-C₆)-cycloalkyl, phenyl, (C₁-C₄)-alkoxy, cyano, (C₁-C₄)-alkylthio, (C₁-C₄)-alkylsulfinyl, (C₁-C₄)-alkylsulfonyl, (C₁-C₄)-alkoxycarbonyl or (C₁-C₄)-alkylcarbonyl,
n is 0, 1 or 2 and
m is 1 or 2.

7. A compound of the formula (I) or a salt thereof as defined in any of claims 1 to 6, with the exception of compounds of the formula (I), where
a) R² =H, R³ = H, n = 0 and
a1)
R¹ = CH₃ and
m = 0 or (R⁵)ₘ =2-, 3- or 4-CH₃, 4-C₂H₅, 4-n-C₃H₇, 4-i-C₃H₇, 4-OCH₃, 4-i-OC₃H₇, 4-NH₂, 4-Cl, 4-NO₂, 2-COOH, 2,3-(CH₃)₂, 2,4-(CH₃)₂, 3,4-(CH₃)₂, 2,5-(CH₃)₂, 2,4,5-(CH₃)₃, 2,4,6-(CH₃)₃, 2,3,4,5,6-(CH₃)₅, 3-CH₃-4-OCH₃, 3-CH₃-4-SCH₃, 2,4-(OCH₃)₂, 2,5-(OCH₃)₂, 3,4,5-(OCH₃)₃, 2-OCH₃-4-NH₂, 2-OCH₃-4-NO₂, 2-OH-4-NO₂ or 2-OH-4-NH₂
a2)
R¹ = H, CH₃, n-C₃H₇, n-C₄H₉, n-C₅H₁₁, n-C₆H₁₃, cyclohexyl, 2-methylphenyl, or n-C₆H₅-CH=CH- and in each case
(R⁵)ₘ = 2-CH₃,
a3)
R¹ = n-C₅H₁₁ and
m = 0 or (R⁵)ₘ = 2-CH₃, 3-NO₂ or 4-NO₂,
a4) R¹ = n-C₉H₁₉ and m = 0,
a5) R¹ = OCH₃, (R⁵)ₘ =2-i-OC₃H₇ or 4-i-OC₃H₇,
a6) R¹ = OC₂H₅, (R⁵)ₘ= 2-OH, 2-OCH₃, 2-COOH, 3,5-(CH₃)₂,
a7)
R¹ = CH₂CH₂COOH and
m = 0 or (R⁵)ₘ= 4-i-OC₃H₇,
a8) R¹ = CH=CHCOOH and (R⁵)ₘ= 2-CH₃ or 4-i-OC₃H₇,
a9) R¹ = 4-methoxyphenyl and (R⁵)ₘ= 4-OCH₃,
a10) R¹ = 4-nitrophenyl and (R⁵)ₘ= 4-NO₂,
a11) R¹ = CH₂ Cl, R²=R⁴=H and m=n=0,
a12)
R¹ = 3,5-dimethyl-1-phenylpyrazol-4-yl or 2,3-dimethyl-1-phenyl-5-oxopyrazol-4-yl and
(R⁵)ₘ= 4-i-OC₃H₇,
a13)
R¹ = C₁₁H₂₃, CH₂Cl, CH₂Br, CH₂l, CHCl₂, CCl₃ or CH₂F and
(R⁵)ₘ = 3,4-(CH₃)₂; see Chem. Abstr. 55: 461g; or
b) R¹ = H, R² =H, R⁴ =CH₃, n= m=0,
c) R¹ = -C(CH₃)=CH₂, R²=R⁴=H, n=0 and (R⁵)ₘ=4-CH₃,
d) R¹ = phenyl, R²=R⁴=H, m=0 and n=0 or (R³)ₙ= 3-phenylcarbonyloxy,
e) N-([(4-benzoylsulfamoyl)phenyl]phthalimide,
f)
R¹=2-(2-carboxyphenyl)phenyl,
R²=R⁴=H, n=0 and m=0 or
(R⁵)ₘ=4-NO₂,
g)
R¹=2-carboxyphenyl, R²=R⁴=H,
n=0 and m=0 or
h) R¹-CH₃, R²=H, R⁴=CH₃ and n=m=0.

8. A process for the preparation of compounds of the formula (I) or salts thereof as defined in claim 7, which comprises
1. reacting a compound of the formula (II) 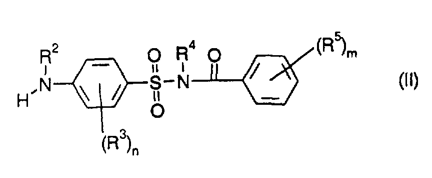 where R², R³, R⁴, R⁵, n and m are as defined in formula (I), with an acylating agent of the formula R¹-CO-Nuc, where Nuc is a leaving group and R¹ is as defined in formula (I), or
2. reacting a compound of the formula (III) 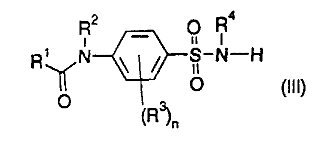 where R¹, R², R³, R⁴ and n are as defined in formula (I) with a benzoyl halide of the formula (IV) 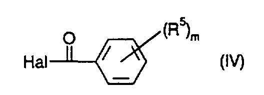 where R⁵ and m have the meanings given in formula (I).

9. A pesticidal composition with an effective content of
A) one or more pesticidally active substances which, when used without safener, may result in phytotoxic damage to crop plants,
B) one or more safeners of the formula (I) or a salt thereof as defined in any of claims 1 to 7.

10. A method of protecting crop plants against phytotoxic side effects of pesticides, which comprises applying, as safener, an effective amount of at least one compound of the formula (I) or a salt thereof, which is as defined in any of claims 1 to 7, before, after or simultaneously with a pesticidally active substance to the plants, the seeds of the plants or the area under cultivation.

## Revendications

1. Utilisation de composés de formule (I) ou de leurs sels comme antidotes pour la protection de plantes de culture contre des effets secondaires phytotoxiques de pesticides : 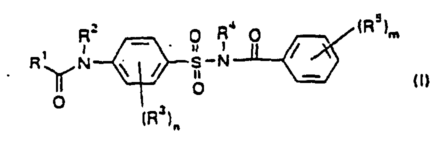 dans laquelle dans la formule (I)
R¹ représente un atome d'hydrogène, un groupe hydrocarboné, un groupe oxyhydrocarboné ou un groupe thiohydrocarboné, dans lequel chacun des 3 derniers groupes cités est non substitué ou substitué par un ou plusieurs groupes identiques ou différents choisis parmi un atome d'halogène, un groupe cyano, nitro, amino, hydroxy, carboxy, formyle, carbonamide, sulfonamide et des groupes de formule -Z^{a}-R^{a},
R² représente un atome d'hydrogène ou un groupe alkyle en C₁ à C₄ ou
R¹ et R² forment ensemble avec le groupe de formule -CO-N- le radical d'un cycle saturé ou insaturé de 3 à 8 chaînons et
R³, dans le cas où n = 1, ou les R³ indépendamment les uns des autres, dans le cas où n est supérieur à 1, représentent un atome d'halogène, un groupe cyano, nitro, amino, hydroxy, carboxy, formyle, CONH₂, SO₂NH₂ ou un groupe de formule -Z^{b}-R^{b},
R⁴ représente un atome d'hydrogène ou un groupe alkyle en C₁ à C₄,
R⁵, dans le cas où m = 1, ou les R⁵ indépendamment les uns des autres, dans le cas où m est supérieur à 1, représentent un atome d'halogène, un groupe cyano, nitro, amino, hydroxy, carboxy, CHO, CONH₂, SO₂NH₂ ou un groupe de formule -Z^{c}-R^{c},
R^{a} représente un groupe hydrocarboné ou un groupe hétérocyclyle, dans lequel chacun des deux derniers groupes cités est non substitué ou substitué par un ou plusieurs groupes identiques ou différents choisis parmi un atome d'halogène, un groupe cyano, nitro, amino, hydroxy, mono- et di-[(alkyl en C₁ à C₄)-amino, ou un groupe alkyle dans lequel plusieurs groupes CH₂ non voisins sont respectivement remplacés par un atome d'oxygène,
R^{b}, R^{c} représentent indépendamment l'un de l'autre un groupe hydrocarboné ou un groupe hétérocyclyle, dans lequel chacun des deux derniers groupes cités est non substitué ou substitué par un ou plusieurs groupes identiques ou différents choisis parmi un atome d'halogène, un groupe cyano, nitro, amino, hydroxy, phosphoryle, halogéno-(alcoxy en C₁ à C₄), mono- et di-[(alkyl en C₁ à C₄)-amino, ou un groupe alkyle, dans lequel plusieurs groupes CH₂ non voisins sont respectivement remplacés par un atome d'oxygène,
Z^{a} représente un groupe divalent de formule -O-, -S-, -CO-, -CS-, -CO-O-, -CO-S-, -O-CO-, -S-CO-, -SO-, -SO₂-, -NR*-, -CO-NR*-, -NR*-CO-, -SO₂-NR*- ou -NR*-SO₂-, dans lesquelles la liaison indiquée à droite des groupes divalents respectifs est la liaison au groupes R^{a} et dans lesquelles les R* dans les 5 derniers groupes cités représentent indépendamment les uns des autres respectivement H, un groupe alkyle en C₁ à C₄ ou halogéno-alkyle en C₁ à C₄,
Z^{b}, Z^{c} représentent indépendamment l'un de l'autre une liaison directe ou un groupe divalent de formule -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*- ou -NR*-CO-, dans lesquelles la liaison indiquée à droite des groupes divalents respectifs est la liaison au groupe R^{b} ou R^{c} et dans lesquelles les R* dans les 5 derniers groupes cités représentent indépendamment les uns des autres respectivement H, un groupe alkyle en C₁ à C₄ ou halogéno-alkyle en C₁ à C₄,
n représente un nombre entier compris entre 0 et 4 et
m un nombre entier entre 0 et 5.

2. Utilisation selon la revendication 1, **caractérisée en ce que**, dans la formule (I)
R¹ représente un atome d'hydrogène, un groupe alkyle en C₁ à C₁₂, alcényle en C₂ à C₈, alcynyle en C₂ à C₈, cycloalkyle en C₃ à C₈, cycloalcényle en C₃ à C₈, alcoxy en C₁ à C₈, alcényloxy en C₂ à C₈, alcynyloxy en C₂ à C₈, cycloalcoxy en C₃ à C₈, cycloalcényloxy en C₃ à C₈, alkylthio en C₁ à C₈, alcénylthio en C₂ à C₈, alcynylthio en C₂ à C₈, cycloalkylthio en C₃ à C₈, cycloalcénylthio en C₃ à C₈ ou aryle, dans lesquels chacun des 16 derniers groupes cités est non substitué ou substitué par un ou plusieurs groupes identiques ou différents choisis parmi un atome d'halogène, un groupe cyano, nitro, amino, hydroxy, carboxy, formyle, carbonamide, sulfonamide et des groupes de formule -Z^{a}-R^{a},
R² représente un atome d'hydrogène ou un groupe alkyle en C₁ à C₄ ou
R¹ et R² forment ensemble avec le groupe de formule -CO-N- le radical d'un cycle saturé ou insaturé de 3 à 8 chaînons, qui en plus de l'atome d'azote du groupe de formule -CO-N- peut encore contenir 1 ou 2 hétéroatomes choisis parmi N, O et S, et
R³, dans le cas où n = 1, ou les R³ indépendamment les uns des autres, dans le cas où n est supérieur à 1, représentent un atome d'halogène, un groupe cyano, nitro, amino, hydroxy, phosphoryle, carboxy, CHO, CONH₂, SO₂NH₂ ou un groupe de formule -Z^{b}-R^{b},
R⁴ représente un atome d'hydrogène ou un groupe alkyle en C₁ à C₄,
R⁵, dans le cas où m = 1, ou les R⁵ indépendamment les uns des autres, dans le cas où m est supérieur à 1, représentent un atome d'halogène, un groupe cyano, nitro, amino, hydroxy, carboxy, phosphoryle, CHO, CONH₂, SO₂NH₂ ou un groupe de formule -Z^{c}-R^{c},
R^{a} représente un groupe alkyle en C₁ à C₆, cycloalkyle en C₃ à C₆, alcényle en C₂ à C₈, cycloalcényle en C₃ à C₆, alcynyle en C₂ à C₈, phényle ou un groupe hétérocyclyle ayant de 3 à 6 atomes de carbone et de 1 à 3 atomes d'hétérocycle choisis parmi N, O ou S, dans lesquels chacun des 7 derniers groupes cités est non substitué ou substitué par un ou plusieurs groupes identiques ou différents choisis parmi un atome d'halogène, un groupe cyano, nitro, amino, hydroxy, mono- et di(alkyle en C₁ à C₄)-amino, ou un groupe alkyle, dans lequel plusieurs groupes CH₂ non voisins peuvent être remplacés respectivement par un atome d'oxygène,
R^{b}, R^{c} représentent indépendamment l'un de l'autre un groupe alkyle en C₁ à C₈, cycloalkyle en C₃ à C₆, alcényle en C₂ à C₈, cycloalcényle en C₃ à C₆, alcynyle en C₂ à C₈, phényle ou un groupe hétérocyclyle ayant de 3 à 6 atomes de carbone et de 1 à 3 atomes d'hétérocycle choisis parmi N, O ou S, dans lesquels chacun des 7 derniers groupes cités est non substitué ou substitué par un ou plusieurs groupes identiques ou différents choisis parmi un atome d'halogène, un groupe cyano, nitro, amino, hydroxy, phosphoryle, halogéno-alcoxy en C₁ à C₄, mono- et di-(alkyle en C₁ à C₄)-amino, ou un groupe alkyle, dans lequel plusieurs groupes CH₂ non voisins peuvent être remplacés respectivement par un atome d'oxygène,
Z^{a} représente un groupe divalent de formule -O-, -S-, -CO-, -CS-, -CO-O-, -CO-S-, -O-CO-, -S-CO-, -SO-, -SO₂-, -NR*-, -CO-NR*- ou -NR*-CO-, dans lesquelles la liaison indiquée à droite des groupes divalents respectifs est la liaison au groupes R^{a} et dans lesquelles les R* dans les deux derniers groupes cités représentent indépendamment les uns des autres respectivement H, un groupe alkyle en C₁ à C₄ ou halogéno-alkyle en C₁ à C₄,
Z^{b}, Z^{c} représentent indépendamment l'un de l'autre une liaison directe ou un groupe divalent de formule -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*- ou -NR*-CO-, dans lesquelles la liaison indiquée à droite des groupes divalents respectifs est la liaison au groupe R^{b} ou R^{c} et dans lesquelles les R* dans les 5 derniers groupes cités représentent indépendamment les uns des autres respectivement H, un groupe alkyle en C₁ à C₄ ou halogéno-alkyle en C₁ à C₄.

3. Utilisation selon la revendication 1 ou 2, **caractérisé en ce que** dans la formule (I)
R¹ représente un atome d'hydrogène, un groupe alkyle en C₁ à C₁₂, alcényle en C₂ à C₈, alcynyle en C₂ à C₈, cycloalkyle en C₃ à C₈, cycloalcényle en C₃ à C₈, alcoxy en C₁ à C₈, alcényloxy en C₂ à C₈, alcynyloxy en C₂ à C₈, cycloalcoxy en C₃ à C₈, cycloalcényloxy en C₃ à C₈, alkylthio en C₁ à C₈, alcénylthio en C₂ à C₈, alcynylthio en C₂ à C₈, cycloalkylthio en C₃ à C₈, cycloalcénylthio en C₃ à C₈ ou phényle, dans lesquels chacun des groupes carbonés précédents cités est non substitué ou substitué par un ou plusieurs groupes identiques ou différents choisis parmi un atome d'halogène, un groupe cyano, nitro, amino, hydroxy, alcoxy en C₁ à C₈, dans lesquels un ou plusieurs groupes CH₂ non voisins peuvent être remplacés par un atome d'oxygène, un groupe alkylthio en C₁ à C₈, alkylsulfinyle en C₁ à C₆, alkylsulfonyle en C₁ à C₆, alcényloxy en C₂ à C₈, alcénylthio en C₂ à C₈, alcynyloxy en C₂ à C₈, alcynylthio en C₂ à C₈, cycloalkyle en C₃ à C₇, cycloalcényle en C₃ à C₇, cycloalcoxy en C₃ à C₇, cycloalcényloxy en C₃ à C₇, mono- et di-(alkyle en C₁ à C₄)-amino, (alcoxy en C₁ à C₈)-carbonyle, (alcényloxy en C₁ à C₈)-carbonyle, (alcynyloxy en C₁ à C₈)-carbonyle, (alkylthio en C₁ à C₈)-carbonyle, (alkyle en C₁ à C₈)-carbonyle, (alcényle en C₂ à C₈)-carbonyle, (alcynyle en C₂ à C₈)-carbonyle, phényle, phényl-alcoxy en C₁ à C₆, hétérocyclyle ayant de 3 à 6 atomes de cycle et de 1 à 3 atomes d'hétérocycle choisis parmi N, O et S et dans le cas de groupes cycliques également un groupe alkyle en C₁ à C₆, chacun des 25 derniers groupes cités étant non substitué ou substitué par un ou plusieurs groupes choisis parmi un atome d'halogène, un groupe nitro, amino, cyano et hydroxy,
R² représente un atome d'hydrogène ou un groupe alkyle en C₁ à C₄ ou
R¹ et R² forment ensemble avec le groupe de formule -CO-N- le résidu d'un cycle hétérocyclique saturé ou insaturé à 5 ou 6 chaînons, qui peut contenir en plus de l'atome d'azote du groupe de formule -CO-N- encore un hétéroatome choisi parmi N, O ou S, et
R³, R⁵ représentent respectivement des groupes identiques ou différents, qui indépendamment l'un de l'autre sont un atome d'halogène, un groupe nitro, amino, hydroxy, cyano, sulfamoyle, alkyle en C₁ à C₈, cycloalkyle en C₃ à C₆, alcényle en C₂ à C₈, alcynyle en C₂ à C₈, alcoxy en C₁ à C₈, alcényloxy en C₂ à C₈, alcynyloxy en C₂ à C₈, mono- et di(alkyle en C₁ à C₄)-aminosulfonyle, alkylthio en C₁ à C₈, alkylsulfinyle en C₁ à C₈, alkylsulfonyle en C₁ à C₈, alcoxycarbonyle en C₁ à C₈, alkylthiocarbonyle en C₁ à C₈, alkylcarbonyle en C₁ à C₈, chacun des 15 derniers groupes cités étant non substitué ou substitué par un ou plusieurs groupes identiques ou différents choisis parmi un atome d'halogène, un groupe halogéno-(alcoxy en C₁ à C₆), phosphoryle, nitro, amino, cyano, hydroxy, alcoxy en C₁ à C₈, dans lesquels un ou plusieurs groupes CH₂ non voisins peuvent être remplacés par un atome d'oxygène, et dans le cas des groupes cycliques est encore substitué par un groupe alkyle en C₁ à C₄ et halogénoalkyle en C₁ à C₄.

4. Utilisation selon l'une des revendications 1 à 3, **caractérisée en ce que** dans la formule (I)
R¹ représente un atome d'hydrogène, un groupe alkyle en C₁ à C₈, alcényle en C₂ à C₈, alcynyle en C₂ à C₈, cycloalkyle en C₃ à C₈, alcoxy en C₁ à C₆, alcényloxy en C₂ à C₆, alcynyloxy en C₂ à C₆, cycloalcoxy en C₃ à C₆, cycloalcényloxy en C₅ à C₆, alkylthio en C₂ à C₆, alcénylthio en C₂ à C₆, alcynylthio en C₂ à C₆, cycloalkylthio en C₃ à C₆, cycloalcénylthio en C₅ à C₆ ou phényle, dans lesquels chacun des 16 derniers groupes cités est non substitué ou substitué par un ou plusieurs substituants identiques ou différents choisis parmi un atome d'halogène, un groupe cyano, alcoxy en C₁ à C₆, alkylthio en C₁ à C₆, alkylsulfinyle en C₁ à C₄, alcényloxy en C₂ à C₄, alcénylthio en C₂ à C₄, alcynyloxy en C₂ à C₄, alcynylthio en C₂ à C₄, cycloalkyle en C₃ à C₆, cycloalcényle en C₅ à C₆, cycloalcoxy en C₃ à C₆, cycloalcényloxy en C₅ à C₆, mono- et di-(alkyle en C₁ à C₄)-amino, (alcoxy en C₁ à C₆)-carbonyle, (alkylthio en C₁ à C₆)-carbonyle, alkyle en C₁ à C₆)-carbonyle, phényle, phényl-alcoxy en C₁ à C₄), hétérocyclyle avec de 5 à 6 atomes de cycle et de 1 à 3 atomes d'hétérocycle choisis parmi N, O et S et dans le cas de groupes cycliques également par un groupe alkyle en C₁ à C₄, dans lesquels chacun des 21 derniers groupes cités est non substitué ou substitué par un ou plusieurs groupes choisis parmi un atome d'halogène et un groupe cyano et dans le cas de groupes cycliques par également un groupe alkyle en C₁ à C₄,
R² représente un atome d'hydrogène ou un groupe alkyle en C₁ à C₄ ou
R¹ et R² forment ensemble avec le groupe de formule -CO-N- le résidu d'un cycle hétérocyclique saturé ou insaturé à 5 ou 6 chaînons, qui ne contient pas d'autre hétéroatome de cycle en plus de l'atome d'azote du groupe de formule -CO-N-, et
R³, R⁵ représentent respectivement des groupes identiques ou différents, qui indépendamment l'un de l'autre sont un atome d'halogène, un groupe nitro, amino, hydroxy, cyano, sulfamoyle, alkyle en C₁ à C₆, cycloalkyle en C₃ à C₆, alcényle en C₂ à C₆, alcynyle en C₂ à C₈, alcoxy en C₁ à C₈, alcényloxy en C₂ à C₆, alcynyloxy en C₂ à C₆, mono- et di(alkyle en C₁ à C₄)-aminosulfonyle, alkylthio en C₁ à C₆, alkylsulfinyle en C₁ à C₈, alkylsulfonyle en C₁ à C₆, alcoxycarbonyle en C₁ à C₆, alkylthiocarbonyle en C₁ à C₆, alkylcarbonyle en C₁ à C₆, chacun des 15 derniers groupes cités étant non substitué ou substitué par un ou plusieurs groupes identiques ou différents choisis parmi un atome d'halogène, un groupe halogéno-(alcoxy en C₁ à C₄), cyano, alcoxy en C₁ à C₆, et dans le cas des groupes cycliques est encore substitué par un groupe alkyle en C₁ à C₄ et halogénoalkyle en C₁ à C₄.

5. Utilisation selon l'une des revendications 1 à 4, **caractérisée en ce que** dans la formule (I)
R¹ représente un atome d'hydrogène, un groupe alkyle en C₁ à C₆, cycloalkyle en C₃ à C₆, alcényle en C₂ à C₆, cycloalcényle en C₅ à C₆ ou phényle, dans lesquels chacun des 6 derniers groupes cités est non substitué ou substitué par un ou plusieurs groupes identiques ou différents choisis parmi un atome d'halogène, un groupe alcoxy en C₁ à C₆, dans lesquels un ou plusieurs groupes CH₂ peuvent être remplacés par l'oxygène, un groupe halogénoalcoxy en C₁ à C₆, alkylsulfinyle en C₁ à C₂, alkylsulfonyle en C₁ à C₂, cycloalkyle en C₃ à C₆, alcoxycarbonyle en C₁ à C₄, alkylcarbonyle en C₁ à C₄ et phényle et dans le cas de groupes cycliques également par un groupe alkyle en C₁ à C₄ et halogénoalkyle en C₁ à C₄,
R² représente un atome d'hydrogène,
R³ représente un atome d'halogène, un groupe halogéno-alkyle en C₁ à C₄, halogéno-alcoxy en C₁ à C₄, alkyle en C₁ à C₄, alcoxy en C₁ à C₄, alkylsulfonyle en C₁ à C₄, alcoxycarbonyle en C₁ à C₄ ou alkylcarbonyle en C₁ à C₄,
R⁴ représente un atome d'hydrogène,
R⁵ représente un atome d'halogène, un groupe alkyle en C₁ à C₄, halogéno-alkyle en C₁ à C₄, halogéno-alcoxy en C₁ à C₄, cycloalkyle en C₃ à C₆, phényle, alcoxy en C₁ à C₄, cyano, alkylthio en C₁ à C₄, alkylsulfinyle en C₁ à C₄, alkylsulfonyle en C₁ à C₄, alcoxycarbonyle en C₁ à C₄ ou alkylcarbonyle en C₁ à C₄,
n est 0, 1 ou 2 et
m est 1 ou 2.

6. Utilisation selon l'une des revendications 1 à 5, **caractérisée en ce que**, dans la formule (I)
R¹ représente un atome d'hydrogène, un groupe alkyle en C₁ à C₆ ou cycloalkyle en C₃ à C₆, dans lesquels chacun des 2 derniers groupes cités est non substitué ou substitué par un ou plusieurs groupes identiques ou différents choisis parmi un atome d'halogène, un groupe alcoxy en C₁ à C₄, halogénoalcoxy en C₁ à C₆ et alkylthio en C₁ à C₄ et, dans le cas de groupes cycliques également par un groupe alkyle en C₁ à C₄ et halogénoalkyle en C₁ à C₄,
R² représente un atome d'hydrogène,
R³ représente un atome d'halogène, un groupe halogéno-alkyle en C₁ à C₄, halogéno-alcoxy en C₁ à C₄, alkyle en C₁ à C₄, alcoxy en C₁ à C₄, alkylsulfonyle en C₁ à C₄, alcoxycarbonyle en C₁ à C₄ ou alkylcarbonyle en C₁ à C₄,
R⁴ représente un atome d'hydrogène,
R⁵ représente un atome d'halogène, un groupe alkyle en C₁ à C₄, halogéno-alkyle en C₁ à C₄, halogéno-alcoxy en C₁ à C₄, cycloalkyle en C₃ à C₆, phényle, alcoxy en C₁ à C₄, cyano, alkylthio en C₁ à C₄, alkylsulfinyle en C₁ à C₄, alkylsulfonyle en C₁ à C₄, alcoxycarbonyle en C₁ à C₄ ou alkylcarbonyle en C₁ à C₄,
n est 0, 1 ou 2 et
m est 1 ou 2.

7. Composés de formule (I) et leurs sels, comme ils sont définis dans une des revendications 1 à 6, à l'exception des composés de formule (I), dans laquelle
a) R² = H, R³ = H, n = 0 et
a1)
R¹ = CH₃ et
m = 0 ou (R⁵)ₘ = 2-, 3- ou 4-CH₃, 4-C₂H₅, 4-n-C₃H₇, 4-OCH₃, 4-i-OC₃H₇, 4-NH₂, 4-NO₂, 2-COOH, 2,3-(CH₃)₂, 2,4-(CH₃)₂, 3,4-(CH₃)₂, 2,5-(CH₃)₂, 2,4,5-(CH₃)₃, 2,4,6-(CH₃)₃, 2,3,4,5,6-(CH₃)₅, 3-CH₃-4-OCH₃, 3-CH₃-4-SCH₃, 2,4-(OCH₃)₂, 2,5-(OCH₃)₂, 3,4,5-(OCH₃)₃, 2-OCH₃-4-NH₂, 2-OCH₃-4-NO₂, 2-OH-4-NO₂ ou 2-OH-4-NH₂,
a2) R¹ = H, CH₃, n-C₃H₇, n-C₄H₉, n-C₅H₁₁, n-C₆H₁₃, un groupe cyclohexyle, 2-méthylphényle ou n-C₆H₅-CH=CH- et respectivement (R⁵)ₘ = 2-CH₃,
a3)
R¹ = n-C₅H₁₁ et
m = 0 ou (R⁵)ₘ = 2-CH₃, 3-NO₂ ou 4-NO₂,
a4) R¹ = n-C₉H₁₉ et m = 0,
a5) R¹ = OCH₃, (R⁵)ₘ = 2-i-OC₃H₇ ou = 4-i-OC₃H₇,
a6) R¹ = OC₂H₅, (R⁵)ₘ = 2-OH, 2-OCH₃, 2-COOH, 3,5-(CH₃)₂,
a7)
R¹ = CH₂CH₂COOH et
m = O ou (R⁵)ₘ = 4-i-OC₃H₇,
a8) R¹ = CH=CHCOOH et (R⁵)ₘ = 2-CH₃ ou 4-i-OC₃H₇,
a9) R¹ = 4-méthoxyphényle et (R⁵)ₘ = 4-OCH₃,
a10) R¹ = 4-nitrophényle et (R⁵)ₘ = 4-NO₂,
a11) R¹ = CH₂Cl, R² = R⁴ = H et m = n = 0,
a12)
R¹ = 3,5-diméthyl-1-phényl-pyrazol-4-yle ou 2,3-diméthyl-1-phényl-5-oxo-pyrazol-4-yle et
(R⁵)ₘ = 4-i-OC₃H₇,
a13) R¹ = C₁₁H₂₃, CH₂Cl, CH₂Br, CH₂I, CHCl₂, CCl₃ ou CH₂F et (R⁵)ₘ = 3,4-(CH₃)₂; voir Chem. Abstr. 55 : 461g ; ou
b) R¹ = H R² = H, R⁴ = CH₃, m = n = 0,
c) R¹ = -C(CH₃) = CH2, R² = R⁴ = H, n = 0 et (R⁵)ₘ = 4-CH₃,
d) R¹ = phényle, R² = R⁴ = H, m = 0 et n = 0 ou (R³)ₙ = 3-phénylcarbonyloxy,
e) N-[(4-benzoylsulfamoyl)phényl]-phtalimide,
f) R¹ = 2-(2-carboxyphényl)phényl, R² = R⁴ = H, n = 0 et m = 0 ou (R⁵)ₘ = 4-NO₂,
g) R¹ = 2-carboxyphényle, R² = R⁴ = H, n = 0 et m = 0 ou
h) R¹ = CH₃, R² = H, R⁴ = CH₃ et n = m = 0.

8. Procédé pour la préparation de composés de formule (I) ou de leurs sels, comme ils sont définis à la revendication 7, **caractérisé en ce qu'**on met à réagir
1. un composé de formule (II), 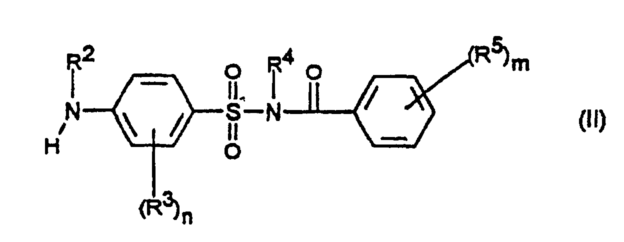 dans laquelle R², R³, R⁴, R⁵, n et m sont définis comme à la formule (I), avec un agent d'acylation de formule R¹-CO-Nuc, dans laquelle Nuc est un groupe partant et R¹ est défini comme à la formule (I), ou on met à réagir un composé de formule (III), 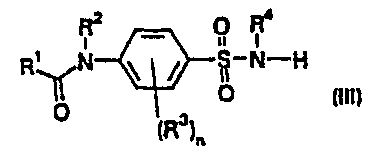 dans laquelle R¹, R², R³, R⁴ et n sont définis comme à la formule (I), avec un halogénure de benzoyle de formule (IV), 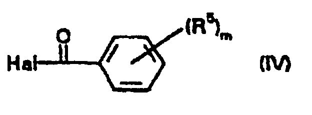 dans laquelle R⁵ et m ont les significations données à la formule (I).

9. Agents pesticides ayant une teneur active en
A) un ou plusieurs substances actives pesticides, qui peuvent produire dans une utilisation sans antidote des dommages phytotoxiques sur les plantes de culture,
B) un ou plusieurs antidotes de formule (I) ou leur sel, comme ils sont définis selon l'une des revendications 1 à 7.

10. Procédé pour la protection de plantes de culture contre les effets phytotoxiques secondaires de pesticides, qui est **caractérisé en ce qu'**on applique une quantité efficace d'au moins un composé de formule (I) ou de son sel, qui est défini selon l'une des revendications 1 à 7, comme antidote avant, après ou en même temps qu'une substance active pesticide sur les plantes, les semences des plantes, ou les surfaces agricoles.
